(19) Europäisches Patentamt
European Patent Office
Office européen des brevets

(11) Numéro de publication: **0 004 823**
**B1**

(12) **FASCICULE DE BREVET EUROPEEN**

(45) Date de publication du fascicule du brevet: **10.08.83**

(21) Numéro de dépôt: **79400233.7**

(22) Date de dépôt: **10.04.79**

(51) Int. Cl.³: **C 07 H 3/08**, C 07 H 3/06, C 07 H 15/18, A 61 K 31/70

(54) **Procédé de préparation de dérivés d'osides, les nouveaux dérivés d'osides obtenus et leurs applications biologiques.**

(30) Priorité: **10.04.78 FR 7810558**

(43) Date de publication de la demande:
**17.10.79 Bulletin 79/21**

(45) Mention de la délivrance du brevet:
**10.08.83 Bulletin 83/32**

(84) Etats contractants désignés:
**BE CH DE GB IT LU NL SE**

(56) Documents cités:
**TETRAHEDRON LETTERS, no. 45, November 1976, J. R. POUGNY et al.: "Réaction d'imidates de glucopyranosyle avec l'acétonitrile. Applications synthétiques", pages 4073—4076**

**L. F. FIESER et al.: "Reagents for organic synthesis", publié 1967, John Wiley & Sons, New York, US Pages 286 et 288**

(73) Titulaire: **ANVAR Agence Nationale de Valorisation de la Recherche**
**13, rue Madeleine Michelis**
**F-92522 Neuilly-sur-Seine (FR)**

(72) Inventeur: **Sinay, Pierre**
**5, rue Jacques Monod**
**F-45100 Orleans (FR)**
Inventeur: **Jacquinet, Jean-Claude**
**1, Allée André Gide**
**F-45100 Orleans-La-Source (FR)**
Inventeur: **Pougny, Jean-René**
**30, rue du Général de Gaulle**
**F-18700 Aubigny-Sur-Mere (FR)**

(74) Mandataire: **Peaucelle, Chantal et al,**
**Cabinet Plasseraud 84, rue d'Amsterdam**
**F-75009 Paris (FR)**

(56) Documents cités:
**JOURNAL OF THE CHEMICAL SOCIETY, Perkin Transactions I; Organic and Bioorganic chemistry, 22, 1977, P. ROLLIN et al.: "Preparation of benzyl ethers of 1,2-dideoxy-2'-methyl-alpha-D-gluco-pyranoso(2,1-d)-delta 2'-oxazoline for use in oligosaccharide synthesis" Page 2514**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 004 823 B1

(56) Documents cités:

JOURNAL OF THE CHEMICAL SOCIETY, Perkin Transactions I; Organic and Bioorganic Chemistry, 11, 1977, C. AUGE et al.: "Studies in oligosaccharide chemistry. Part 8. Synthesis of lacto-N-triose I, a core chain trisaccharide of human blood-group substances" Page 1343

JOURNAL OF THE CHEMICAL SOCIETY, Perkin Transactions I; Organic and Bioorganic chemistry, 4, 1975, P. A. GENT et al.: "The allyl ether as a protecting group in carbohydrate chemistry. Part VII. The 2-O-allyl group as a non-participant in 1,2-cis-glucoside synthesis" Page 361

JOURNAL OF THE AMERICAN CHEMICAL SOCIETY, 97:14, juillet 1975, R. U. LEMIEUX et al.: "The chemical synthesis of 2-O-(alpha-L-fuco-pyranosyl)-3-O-(alpha-D-galacto-pyranosyl)-D-galactose. The terminal structure of the blood-group B antigenic determinant", pages 4069—4075

THE JOURNAL OF ORGANIC CHEMISTRY, vol. 42, no. 4, 18. février 1977, J. C. JACQUINET et al.: "Synthesis of blood-group substances 6. Synthesis of O-alpha-L-fucopyranosyl-(1-2)-O-beta-D-galactopyranosyl-(1-4)-O-(alpha-L-fucopyranosyl-(1-3)-2-acetamido-2-deoxy-alpha-D-glucopyranose the postulated Lewis d antigenic determinant", pages 720—724

CHEMICAL ABSTRACTS, vol. 87, no. 3, 18 juillet 1977, résumé nr. 23639a, Columbus, Ohio, US, C. AUGE et al.: 'Studies in oligosaccharide chemistry; Part VII. Syntheses of 3-O(2-acetamido-2-deoxy-beta-D-glucopyranosyl)-alpha-D-galactopyranose ("Lacto-N-biose II") and 3,4-di-O-(2-acetamido-2-deoxy-beta-D-glucopyranosyl)-D-galactopyranose" Page 690

JOURNAL OF THE CHEMICAL SOCIETY, Perkin Transactions; Organic and Bioorganic Chemistry, 18, 1975, P. A. GENT et al.: "Synthesis of 3-O-(6-O-(6-O-(alpha-D-galactopyranosyl)-alpha-D-galactopyranosyl)-beta-D-galactopyranosyl)-1,2-di-O-stearoyl-L-glycerol, a 'Trigalactosyl diglyceride' ", pages 1779—1781

# Procédé de préparation de dérivés d'osides, les nouveaux dérivés d'osides obtenus et leurs applications biologiques

L'invention a pour objet un nouveau procédé de préparation de dérivés d'osides, les nouveaux dérivés d'osides obtenus et leurs applications biologiques.

L'étude des dérivés d'osides, c'est-à-dire de dérivés comportant plusieurs motifs oses ou monosaccharides, suscite un grand intérêt: un certain nombre d'entre eux se sont, en effet, révélés biologiquement et chimiquement actifs ou capables de conduire à de telles substances actives.

Entre autres, il s'est avéré que certains tri-et tétrasaccharides possèdent une spécificité de groupe sanguin.

Ainsi, le trisaccharide 2-O-(*α-L*-fucopyranosyl)-3-O-(*α-D*-galactopyranosyl)-*D*-galactopyranose de formule I:

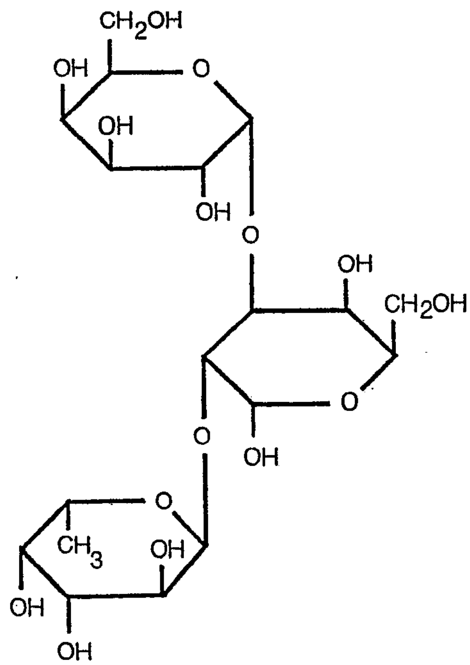

(I)

constitue le déterminant antigénique B de groupe sanguin humain. En raison de cette spécificité antigénique, ce trisaccharide (appelé ci-après trisaccharide B) trouve des applications de grande importance dans divers secteurs biologiques, en particulier en immunohématologie et plus spécialement dans la détermination des groupes sanguins.

Mais ces diverses applications ne peuvent être mises en oeuvre à grande échelle que si l'on peut disposer aisément et avec des rendements élevés du trisaccharide B ou de dérivés osidiques possédant sa structure.

Outre le problème de la synthèse de ces produits, se pose celui de la forme sous laquelle ils sont obtenus. On remarquera à cet égard que, selon le type d'application envisagé, le dérivé d'oside à spécificité antigénique pourra être utilisé tel quel, par exemple comme réactif antigénique ou pour élaborer un sérum-test ou devra comporter une chaîne de substitution, plus particulièrement un bras de couplage afin d'être fixé sur un support, et ce, pour constituer plus spécialement un antigène artificiel ou un immunoabsorbant.

Le problème est donc de disposer d'un dérivé osidique à partir duquel les diverses applications souhaitées pourront être aisément mises en oeuvre.

Or, les méthodes proposées à ce jour pour la synthèse de dérivés osidiques constituant les déterminants antigéniques de groupe sanguin et les rendements auxquels elles conduisent ne s'avèrent pas entièrement satisfaisants.

De plus, les produits obtenus ne peuvent être facilement modifiés en vue de leur utilisation dans une application donnée.

L'étude de ces problèmes par les inventeurs les a conduits à l'elaboration d'une nouvelle voie de synthèse stéréospécifique permettant d'obtenir aisément, avec une excellente pureté et des rendements élevés, des dérivés d'osides ouvrant la voie à de nombreuses applications biologiques. D'une manière particulièrement avantageuse, cette voie de synthèse permet d'obtenir des osides branchés, c'est-à-dire à chaînes osidiques latérales, tel que le trisaccharide B.

L'intérêt de cette nouvelle voie de synthèse est d'autant plus précieux qu'elle conduit à l'obtention

de nouveaux dérivés d'osides, en particulier de dérivés de substitution du trisaccharide B utilisables notamment dans les applications biologiques évoquées plus haut.

D'une manière avantageuse, il s'avère que ces nouveaux dérivés, grâce à leurs caractéristiques chimiques propres, facilitent la mise en oeuvre de ces applications, en particulier peuvent être aisément modifiés selon l'application souhaitée ou même directement utilisables.

L'invention a donc pour but de fournir un nouveau procédé de synthèse de dérivés d'osides permettant de disposer aisément de grandes quantités d'osides et notamment d'osides branchés tels que le trisaccharide B ou des dérivés de substitution de ce dernier.

Selon un autre aspect, elle a pour but de fournir de nouveaux osides, en particulier de nouveaux dérivés de trisaccharides, et plus spécialement des dérivés d'osides possédant la structure du trisaccharide B.

Selon encore un autre aspect, elle vise les applications biologiques des nouveaux dérivés d'osides.

Le procédé de synthèse de dérivés d'osides selon l'invention est caractérisé en cequ'on fait réagir

—un dérivé d'oside a) constitué par un ou plusieurs motifs oses, éventuellement rattaché (s) à un reste organique, ce motif ose, ou l'un au moins de ces motifs oses, étant substitué, sur le carbone anomère en position 1, par un groupe —O— imidyle de formule $—O—C(—R_2)=N—R_1$ dans laquelle les substituants $R_1$ et $R_2$, identiques ou différents l'un de l'autre, représentent un radical alcoyle, de préférence un radical alcoyle comprenant 1 à 4 atomes de carbone, les groupes —OH du dérivé d'oside étant protégés par des groupes appropriés, avec

—un derivé d'oside (b) constitué par un ou plusieurs motifs oses, éventuellement rattaché (s) à un reste organique, un seul groupe —OH de ce ou ces motifs oses étant libre et occupant l'une quelconque des positions hydroxyle secondaire 1 à 4, les dérivés d'osides a) et/ou b) comprenant un motif avec au moins deux oses.

Un procédé de ce type est avantageusement mis en oeuvre pour la synthèse de dérivés comprenant des osides branchés.

A cet effet, on procède à la réaction du dérivé a) défini plus haut avec un dérivé d'oside constitué par un ou plusieurs motifs oses, éventuellement rattaché (s) à un reste organique, un seul groupe —OH de ce ou ces motifs étant libre et occupant une position hydroxyle 1 à 4 ou 6 permettant la formation d'une chaîne osidique branchée.

Cette réaction est avantageusement utilisable pour la synthèse d'un dérivé d'oside constitué par un trisaccharide branché ou comprenant, rattaché par exemple à un reste organique, un tel trisaccharide branché.

Selon un mode préféré de réalisation de l'invention qui ouvre la voie notamment à l'obtention de substances à propriétés antigéniques, on met en oeuvre, dans les diverses formes d'exécution du procédé ci-dessus, un dérivé d'oside a) constitué par un motif galactopyranose, ou un motif fucopyranose, substitué en position 1 par un groupe O-imidyle tel que défini ci-dessus.

Suivant une disposition complémentaire, le dérivé d'oside b) comprend un motif ose comportant en position 1 une substitution —OA constituée par un groupe fonctionnel, non réactif dans les conditions de la synthèse osidique, ou rendu non réactif, ou par un groupe fonctionnalisable, c'est-à-dire autorisant au cours ou en fin de synthèse l'introduction de groupements fonctionnels.

Il s'agit par là essentiellement de disposer en position 1 d'un groupe permettant de mettre aisément à profit les propriétés des osides obtenus et ce dans les diverses applications biologiques mettant en jeu les propriétés des déterminants antigéniques.

Des groupes *A* particulièrement appropriés sont constitués par des radicaux comportant une ou plusieurs liaisons insaturées, tels que les radicaux éthyléniquement insaturés. De préférence, *A* représente alors un radical alcényle comprenant de 2 à 10 atomes de carbone.

*A* représentera également des radicaux auxquels peuvent donner lieu, selon les techniques classiques de synthèse organique, la fonctionnalisation de la ou des liaisons insaturées en question. Des radicaux de ce dernier type sont, par exemple, constitués par des radicaux alcoyle, comportant notamment 2 à 10 atomes de carbone, et substitués par au moins un groupe —OH qui, le cas échéant, est protégé par un groupe de blocage ou engagé dans un radical fonctionnel.

*A* sera également constitué par un groupe fonctionnel renfermant au moins un radical éther et/ou amine.

Plus spécialement, il est avantageux de mettre en oeuvre dans le procédé de l'invention des dérivés d'osides b) dans lesquels le groupe hydroxyle en position 1 est substitué par un radical O-allyle, étant donné que ce groupement ouvre la voie à l'introduction d'un grand nombre de groupements chimiques.

Des dérivés b) répondant aux caractéristiques ci-dessus, comprenant un motif galactopyranose et/ou fucopyranose sont particulièrement préférés pour la réalisation des produits de l'invention.

Pour préparer, conformément au mode de réalisation préféré ci-dessus, un dérivé de trisaccharide branché comprenant un enchaînement galactopyranose-galactopyranose-fucopyranose et répondant plus spécialement à la structure II:

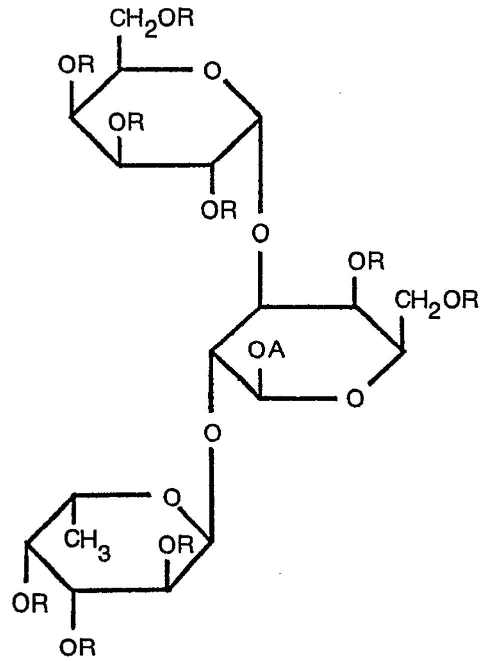

(II)

on fait réagir

—un 1-O-imidyl-*β*-*D*-galactopyranose de formule III:

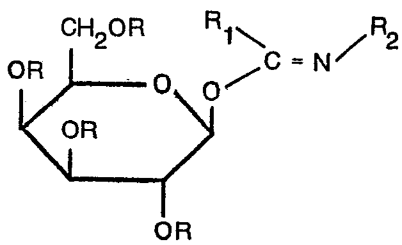

(III)

avec un disaccharide constitué par un fuco-galactopyranose comportant un groupement —OH libre en position 3 du motif galactopyranose, ce disaccharide répondant à la formule IV:

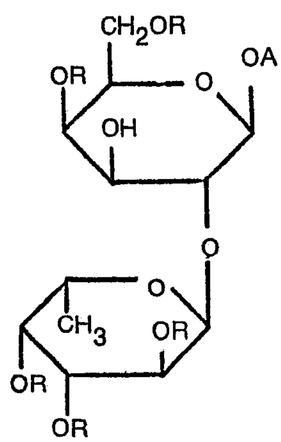

(IV)

Dans ces formules:

—les substituants *R,* identiques ou différents les uns des autres, représentent des groupements de protection de radicaux hydroxyle, le cas échéant avec un substituant voisin, et sont choisis parmi des groupes stables, non réactifs dans les conditions habituelles de synthèse osidique et facilement éliminables dans des conditions douces compatibles avec le maintien de la structure osidique, en particulier par des groupes constituant avec l'atome d'oxygène de l'hydroxyle des éthers benzyliques ou des acétals benzylidéniques,

—*A* représente un reste organique tel que défini ci-dessus, et

—$R_1$ et $R_2$ présentent les significations données plus haut.

De préférence, le substituant *A* du disaccharide de formule IV est constitué par un radical éthyléniquement insaturé choisi parmi les radicaux alcényle, de préférence, comportant de 2 à 10 atomes de carbone. *A* peut être également constitué par un radical alcoyle substitué par au moins un groupe —OH qui, le cas échéant, est protégé par un groupe de blocage ou engagé dans un radical fonctionnel.

D'une manière particulièrement préférée, on a recours à des groupes *R* représentant un radical benzyle et, pour les positions 4 et 6, un radical benzylidène, et l'on choisit comme substituant *A* un radical allyle.

Le choix de ces significations pour *R* et *A* offre la possibilité d'éliminer *A* indépendamment de *R* ou de le traiter, sans que *R* ne soit touché de le modifier en un groupement permettant l'utilisation du dérivé dans une application biologique donnée. Ainsi, on peut transformer le groupe allyle représenté par *A*, notamment, en un groupe β-hydroxyéthyle par exemple sous l'action de tétroxyde d'osmium et de périodate de sodium, dont l'intérêt dans les applications biologiques des produits formés sera souligné ci-après. On dispose ensuite de toute latitude pour éliminer les groupes *R* lorsqu'on le souhaite.

Parmi les larges possibilités de traitement du groupe allyle, on mentionnera encore, à titre d'exemple, sa transformation par hydroboration en un groupe gammahydroxypropyle.

De surcroît, de telles chaînes peuvent être traitées pour l'introduction d'un groupement allyle permettant donc l'articulation d'un grand nombre de réactions et la création de chaînes de substitution à caractère hydrophile, précieuses pour les applications recherchées.

Selon une variante, pour préparer le dérivé de trisaccharide de formule II, on met en oeuvre, comme disaccharide, un digalactopyranose, comportant un groupement —OH libre en position 2 de l'un des motifs galactopyranose, et répondant à la formule:

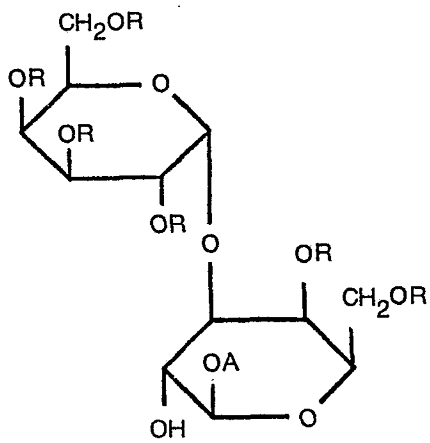

(V)

et on effectue sa condensation avec le groupe -O-imidyle d'un 1-O-imidyl-β-*L*-fucopyranose de formule VI:

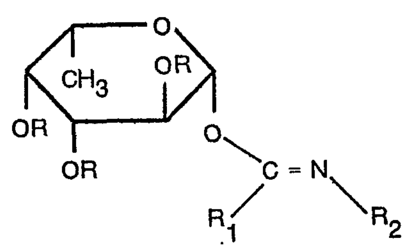

(VI)

De ces formules V et VI, *R*,$R_1$,$R_2$ et *A* présentent les significations données plus haut.

La mise en oeuvre de ce procédé de synthèse conduit à l'obtention, avec un rendement élévé, de motifs trisaccharides possédant la stéréospécificité souhaitée.

Les condensations du procédé de l'invention sont avantageusement réalisées dans des conditions anhydres et à température ambiante.

On opérera en présence d'un acide fort, peu nucléophile et dans un solvant faiblement nucléophile et inerte vis-à-vis de produits réactionnels.

De manière avantageuse, on a recours, comme acide, à de l'acide p-toluène sulfonique anhydre.

Quant au solvant, il est avantageusement constitué par du benzène, de l'éther et plus spécialement par du nitrométhane.

Les disaccharides mis en oeuvre dans les réactions de condensations ci-dessus sont avantageusement préparés selon un procédé relevant du même principe que ces condensations.

Ainsi, pour obtenir le fuco-galactopyranose de formule (IV), on faite avantageusement réagir un galactopyranose comportant un groupe —OH libre en position 2 et répondant à la formule VII:

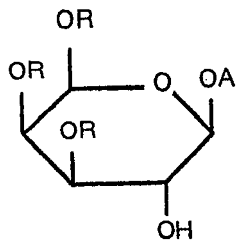

(VII)

avec un fuconvranose de formule VI donnée ci-dessus, puis on procède à l'élimination du groupement *R* en position 3, dans le motif ose provenant du galactopyranose VI, selon les techniques classiques pour le groupe de blocage représenté par R.

De même, le digalactopyranose de formule V est avantageusement obtenu par réaction du galactopyranose de formule VIII, comportant un groupe —OH libre en position 3,

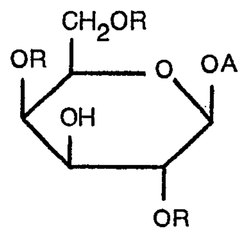

(VIII)

avec un 1-O-imidyl-galactopyranose de formule III ci-dessus. Le disaccharide ainsi obtenu est ensuite traité, selon les méthodes classiques, de manière à éliminer le groupe *R* en position 2 dans le motif ose provenant du galactopyranose VIII.

Selon un aspect de l'invention evêtant un grand intérêt, le procédé ci-dessus permet la synthése totale du trisaccharide B.

A cette fin, après avoir effectué les condensations entre le disaccharide de formule IV ou V et, respectivement, le monosaccharide de formule III ou VI, on procéde à l'élimination séquentielle, dans des conditions douces selon les techniques classiques, du substituant *A* du produit de structure II obtenu, ce qui conduit à un produit de structure IX:

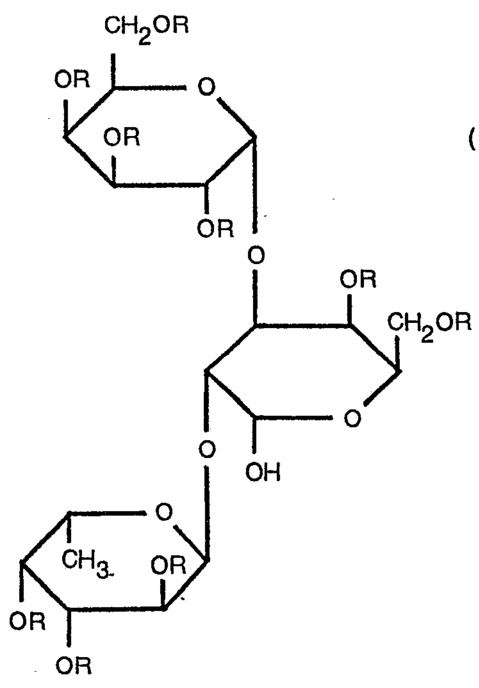

(IX)

dont l'hydrogénolyse permet d'obtenir le trisaccharide B.

Chacune des étapes du procédé de synthése de l'invention se caractérise par un rendement élevé. Leur mise en oeuvre permet ainsi d'obtenir des quantités importantes de trisaccharide B ou de trisaccharides substitués.

La mise au point du procédé évoqué ci-dessus conduit, selon un autre aspect de l'invention de grand intérêt, à l'élaboration de nouveaux dérivés d'osides.

L'étude de ces nouveaux dérivés a montré qu'ils constituent, grâce à leurs caractéristiques chimiques, des substances de synthèse analogues aux antigènes B de groupe sanguin; d'une manière avantageuse, ces dérivés ouvrent également la voie à l'obtention de produits antigéniques.

Les nouveaux osides de l'invention comportent au moins un motif galactopyranose dans lequel l'atome d'hydrogène du groupe —OH en position 1 est substitué par un radical *A* représentant un groupe alcoyle substitué par au moins un groupe hydroxyle, le cas échéant protégé par un groupe de blocage ou engagé dans un radical fonctionnel, ou encore un groupe comportant une ou plusieurs liaisons insaturées, en particulier un radical éthyléniquement insaturé choisi parmi les radicaux alcényle, et plus spécialement les radicaux alcényle à 2 à 10 atomes de carbone, *A* pouvant comporter, en outre, un ou plusieurs groupements éther et/ou amine.

Dans un groupe préféré de dérivés d'osides selon l'invention, *A* représente un radical alcoyle comportant 2 à 10 atomes de carbone, substitué par au moins un groupe hydroxyle, et avantageusement choisi parmi les radicaux alcoylèneglycol ou hydroxyalcoyle.

Des produits dans lesquels *A* représente un groupe $\alpha$-$\beta$ dihydroxypropyle, $\beta$-hydroxyéthyle ou $\delta$-hydroxypropyle constituent des réactifs biologiques et sont également spécialement intéressants dans les applications biologiques faisant intervenir leur fixation sur une protéine ou un support insoluble, par exemple, pour la constitution d'immunoabsorbants.

Dans un autre groupe préféré de dérivés d'osides selon l'invention, *A* représente un radical alcényle comportant 2 à 10 atomes de carbone, en particulier un radical allyle. La réactivité de ce radical s'avère tout spécialement avantageuse dans la conduite de la synthèse osidique proprement dite, dans la mesure où *A* peut être traité indépendamment des groupes de blocage utilisés pour les groupements hydroxyle des motifs oses. L'intérêt de la signification allyle de *A* réside également dans le jeu de possibilités offert pour l'introduction d'un groupement fonctionnel donné selon les applications envisagées. De plus, on notera que le dérivé allylique peut être utilisé tel quel comme réactif biologique par exemple pour neutraliser les hémolysines.

D'autres produits préférés de l'invention comportent un groupement *A* comprenant au moins une fonction ether et au moins un groupement éthyléniquement insaturé avantageusement un groupement allyle. Dans ce groupe de produits, *A* peut représenter, par exemple une chaîne alcoxy-alcényle et notamment alcoxy-allyle, le groupe alcoxy comprenant un nombre variable d'atomes de carbone, généralement inférieur à 10 et plus spécialement de l'ordre de 3.

Dans un autre groupe de produits, *A* est une chaîne de substitution du type alcoxy-alcool, alcoxy-ester, amide ou -amine ou alcoyle-amide, ou alcoxy-acide carboxylique, ces différents groupes chimiques pouvant comporter, en outre, une fonction amine intercalaire.

Les motifs galactopyranose définis ci-dessus sont avantageusement impliqués dans une chaîne trisaccharide linéaire ou branchée comprenant un motif fucopyranose et un autre motif galactopyranose.

Dans ces chaînes trisaccharides, les groupements hydroxyle des différents motifs oses sont libres ou protégés par des groupes de blocage choisis parmi des groupes stables, non réactifs dans les conditions habituelles de synthèse osidique et facilement éliminables dans des conditions douces compatibles avec le maintien de la structure osidique.

Les trisaccharides répondant aux charactéristiques définies ci-dessus, et plus spécialement les trisaccharides branchés constitués d'un motif galactopyranose, comportant un substituant *A* tel qu'évoqué ci-dessus, simultanément substitué par un autre motif galactopyranose et par un motif fucopyranose sont préférés.

En particulier, l'invention vise les trisaccharides branchés de formule II

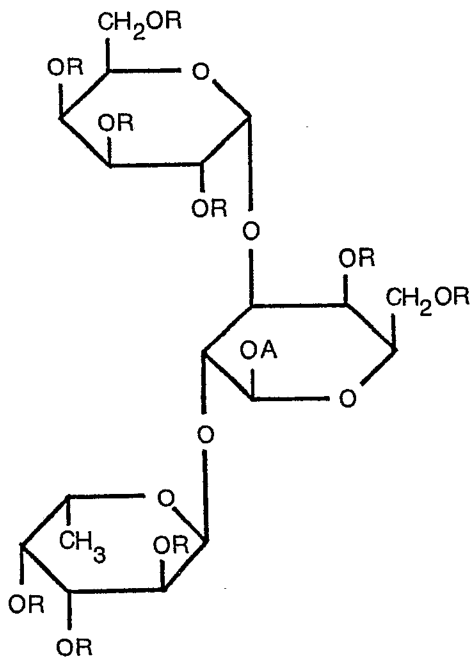

(II)

dans laquelle:

—les substituants *R*, identiques ou différents les uns des autres, représentent, le cas échéant, avec un substituant voisin des groupes de protection de radicaux hydroxyle et sont choisis parmi des groupes stables, non réactifs dans les conditions habituelles de synthèse osidique et facilement éliminables, dans des conditions douces compatibles avec le maintien de la structure osidique, en particulier par des groupes benzyle ou benzylidényle, ou représentent un atome d'hydrogène;

—*A* représente un reste organique comportant au moins un groupe fonctionnalisable et constitué par un radical éthyléniquement insaturé choisi parmi les radicaux alcényle, de préférence de 2 à 10 atomes de carbone, et plus particulièrement constitué par le radical allyle, ou encore par un radical hydroxyalcoyle, plus spécialement $\beta$ ou $\delta$ hydroxyalcoyle dans lequel le groupe —OH est éventuellement protégé ou un radical alcoxy-alcényle alcoxy-alcool, ou -amine ou -amide alcoxy, alcoxy alcoyl-amine ou amide ou alcoxy-acide carboxylique, ces différents groupes chimiques pouvant comporter en outre une fonction amine intercalaire.

Comme indiqué ci-dessus, ces nouveaux osides sont eux-mêmes ou constituent des intermédiaires de grand intérêt pour l'obtention de produits à propriétés de groupes sanguins, en particulier du trisaccharide B ou des produits comportant sa structure.

L'invention fournit donc les moyens permettant d'obtenir des hcmologues de synthèse d'antigène B. Elle fournit également des produits qui constituent de tels homologues. Les trisaccharides de formule II dans laquelle R est un atome d'hydrogène s'avèrent en effet particulièrement précieux à cet égard.

La possibilité de disposer, grâce au procédé de l'invention, des osides en question sous une forme extrêmement pur confère encore plus d'intérêt à leurs propriétés, en particulier dans leurs applications biologiques, notamment en immunohématologie et plus spécialement pour la détermination de groupes sanguins.

On mesurera tout l'intérêt de disposer, par exemple, au niveau de la pratique transfusionnelle, de tels produits de synthèse en considérant le processus utilisé à ce jour pour la détermination de groupes sanguins.

Selon les techniques les plus courantes, ces déterminations s'effectuent à l'aide de sérums-tests d'origine humaine ou produits par immunisation d'animaux.

Pour effectuer la détermination systématique du groupe Rhésus standard (antigène D), plusieurs milliers de litres d'antisérum anti-D sont utilisés chaque année en France. Pour que la spécificité de ce réactif d'origine humaine soit dirigée uniquement contre l'antigène D, il est nécessaire d'absorber les anticorps naturels anti-A et anti-B, initialement présents dans ces sérums, sur des globules rouges A ou B de groupe Rhésus négatif. Plusieurs milliers de litres de sang du groupe B Rhésus négatif sont utilisés à cet effet. Ceci représente non seulement des manipulations de très gros volumes, mais surtout entraîne l'utilisation d'un sang relativement rare.

Ainsi, en France, 1,2% de la population seulement appartient au groupe B Rhésus négatif. Ceci a comme conséquence de provoquer une "pénurie", créée artificiellement, de flacons de sang de ce groupe destinés à la transfusion sanguine.

Les nouveaux trisaccharides de formule II de l'invention dans lesquels *R* représente un atome d'hydrogène constituent précisément une solution à ce problème de "pénurie" de flacons de sang

appartenant à des groupes précieux pour la transfusion sanguine.

En effet, par fixation sur un support solide de ces nouveaux trisaccharides, dotés d'une capacité d'absorption élevée, l'invention fournit un modèle artificiel, qui peut être régénéré, des globules B Rhésus négatif.

Le même type de composé peut être utilisé pour résoudre d'autres problèmes où intervient une fréquence encore plus rare de combinaisons de systèmes de groupe sanguin.

Par exemple, certains sujets appartiennent au groupe exceptionnel négatif pour l'antigène cellano(système Kell) et sont susceptibles de développer un anticorps anticellano pouvant être utilisé comme sérum test pour l'antigène cellano. Selon le processus de détermination classique de groupes sanguins, les anticorps naturels anti-B de ces sujets sont absorbés à l'aide de globules rouges d'un sujet de groupe B, cellano négatif. Si l'on considère qu'une telle combinaison ne se trouve que 1,7 fois sur 10.000, on mesure l'intérêt indéniable de l'invention.

Il est clair que l'utilisation à la place de ces globules rouges, pour jouer le même rôle, de l'immunoabsorbant de l'invention défini ci-dessus présente un intérêt tout particulier.

De plus, il est possible de disposer de sérums-tests spécifiques, grâce aux immunoabsorbants de l'invention dotés d'une capacité d'absorption d'anticorps élevés, inversement, après l'élution en milieu acide des anti-B fixés sur les immunoabsorbants et concentration, on récupère avec des titres élevés les anticorps anti-B, ce qui augmente l'intérêt économique des produits de l'invention.

Dans ces applications, on pourra avantageusement avoir recours aux supports utilisés habituellement dans ce type de technique et plus spécialement à des polymères insolubles tels que de la cellulose, de l'agarose ou des dérivés de ces composés.

D'une manière générale, les osides de l'invention plus spécialement les trisaccharides de formule II définie ci-dessus, sont utilisables in vitro et in vivo grâce à leur innocuité et à leur non immunogénécité, et ce dans les diverses réactions immunologiques mettant en jeu les propriétés du déterminant antigénique B.

L'invention fournit ainsi de précieux réactifs biologiques utilisables sous forme immobilisée, mais également tels quels. L'étude de ces dérivés sous leur forme libre a montré notamment leur capacité élevée de neutralisation des hémolysines anti-B. Ces dérivés sont donc avantageusement utilisables pour la détection et la neutralisation des hémolysines anti-B en remplacement des substances de groupes sanguins isolées à partir de mucine d'estomac de porc ou de cheval.

D'autres caractéristiques et avantages de l'invention apparaîtront dans la suite de la description donnée dans les exemples.

Les points de fusion donnés dans ces exemples sont mesurés dans un tube capillaire au moyen d'un appareil Büchi et ne sont pas corrigés. Les pouvoirs rotatoires sont déterminés avec, comme polarimètre, le modèle 141 commercialisé par Perkin-Elmer. Les spectres infrarouge (IR) sont enregistrés à l'aide d'un spectrophotomètre IRA—1 de Jouan-Jasco et les spectres de résonance mangétique nucléaire (RMN) à l'aide d'un spectromètre Perkin-Elmer R—32 (90MHz). En ce qui concerne les résultats relatifs à la RMN on indique les déplacements chimiques ($\delta$) par rapport au tétraméthylsilane interne; les protons de l'unité *L* fucopyranose sont affectés d'un indice prime, ceux de l'unité *D*-galactopyranose non réductrice d'un indice seconde. Les chromatographies sur colonne sont effectuées au moyen de gel de silice Merck (granulométrie 0,063—0,200 mm).

Exemple 1

Préparation du trisaccharide allyl - 2 - O - (2,3,4 - tri - O - benzyl - $\alpha$ - *L* - fucopyranosyl) - 3 - O - (2,3,4,6 - tetra - O - benzyl - $\alpha$ - galactopyranosyl) - 4,6 - O - benzylidène - $\beta$ - *D* - galactopyranoside de formule X;

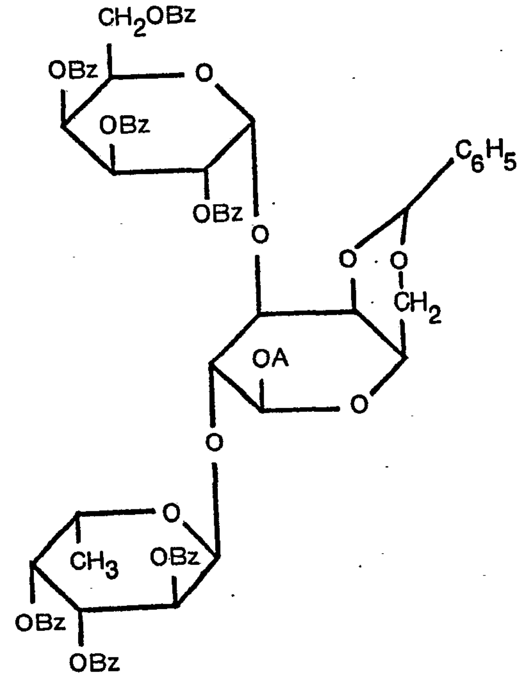

(X)

dans laquelle Bz représente le radical benzyle.

Pour élaborer le trisaccharide de formule X, on fait réagir un disaccharide, à savoir un fucogalactopyranose de forme XI, l'allyl - 3 - O - benzoÿl - 2 - O - (2,3,4 - tri - O - benzyl - $\alpha$ - *L* - fucopyranosyl) - 4,6,O - benzylidène - $\beta$ - *D* - galactopyranoside.

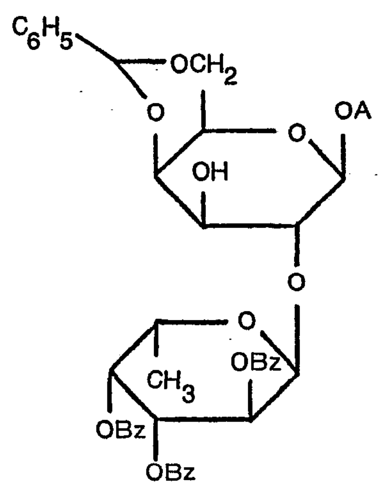

(XI)

avec le 1-O-imidyl-galactopyranose de formule XII (1-O-(N-méthyl)-acétimidyl-2,3,4,6-tétra-O-benzyl-$\beta$-*D*-galactopyranose).

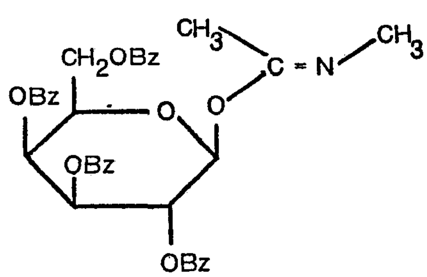

(XII)

Préalablement à cette réaction de condensation, on procède à la préparation du disaccharide XI d'une part, et du 1-O-imidyl galactopyranose XII, d'autre part, en opérant comme suite.

1. Préparation du disaccharide XI.

Le disaccharide XI est obtenu par condensation du fucopyranose de formule XIII (le 1-O(N-méthyl)-acétimidyl-2,3,4-tri-O-benzyl-*β*-*L*-fucopyranose)

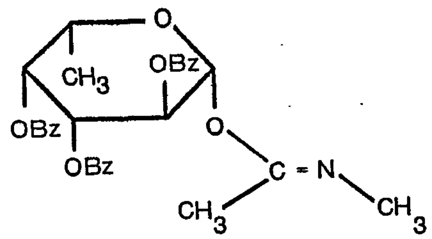

(XIII)

avec l'allylgalactopyranose de formule XIV (l'allyl-3-O-benzoyl-4,6-O-benzylidène-*β*-*D*-galactopyranoside)

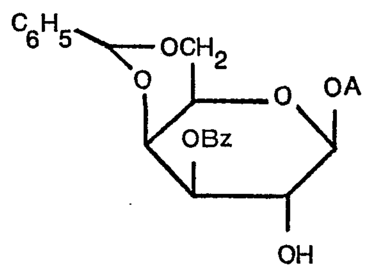

(XIV)

a) Préparation du 1-O-(N-méthyl)-acétimidyl-2,3,4-tri-O-benzyl-*β*-*L*-fucopyranose de formule XIII.

On dissout 434 mg de 2,3,4-tri-O-benzyl-*α*-*L*-fucopyranose de formule XV

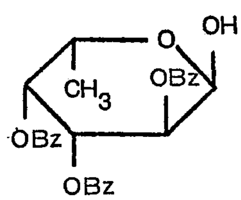

(XV)

dans 10 ml de dichlorométhane puis on ajoute 2,5 équivalents de chlorure de diméthylchloroformiminium. Le mélange réactionnel est soumis à agitation à l'abri de l'humidité puis est filtré au bout de 40 mn, sur une courte colonne de gel de silice et évaporé. On obtient du chlorure de 2,3,4-tri-O-benzyle-*α*-*L*-fucopyranosyle (rdt 96%), qui est engagé directement dans la fabrication du 1-O-(N-méthyl)-acétimidyl fucopyranose.

On ajoute 434 mg de chlorure de fucopyranosyle et 190 mg d'éthyldiisopropylamine à 10 ml d'une solution benzénique anhydre, renfermant 81 mg de N-méthylacétamide, agitée sous azote sec et à l'abri de la lumière, en présence de 580 mg d'oxyde d'argent et de tamis moléculaires 4 Å. Au bout de 20 h, le milieu réactionnel est filtré sur lit d'alumine neutre, lequel est lavé avec 250 mg d'éther contenant 0,1% de triéthylamine. Le filtrat et la phase éthérée sont évaporés à sec et le résidu solide (462 mg, 94%) est cristallisé dans l'hexane, donnant l'imidate recherché (431 mg, 88%).

P.F. 89—90°C; $[\alpha]_D^{20} = -67°$ (*c* 1,benzène); R.M.N. (chloroforme-*d*): *δ* 1,18 (3H, d, 7Hz, Me-C), 1,84 (3H, s, Me-C), 2,97 (3H, s, Me-N), 5,80 (1H, d, $J_{1,2}$ 8Hz, H-1), 7,30—7,35 (15H, m, Ph); anal; calc. pour $C_{30}H_{35}NO_5$: C. 73,59: H: 7,20; N: 2,86; O: 16,34; trouvé: C: 73,72; H: 7,33; N: 2,92; O: 16,19.

b) Préparation de l'allyl-galactopyranose de formule XIV

Elle est réalisée par action d'aldéhyde benzoique en présence de chlorure de zinc sur de l'allyl-*β*-*D*-galactopyranoside, ce qui conduit à l'allyl-4,6-O-benzylidène-*β*-*D*-galactopyranoside qu'on soumet à une réaction de benzoylation. Ces étapes sont effectuées comme suit:

*α*) allyl-*β*-D-galactopyranoside.

On soumet à agitation un mélange de 6 g d'oxyde mercurique, de 0,4 g de bromure mercurique et de 10 g de driérite pendant 230 mn à la température ambiante et à l'abri del'humidité en présence de 80 ml de 1,2-dichloroéthane et d'alcool allylique fraîchement distillé. 12 g de bromure de 1,2,3,4-tétra-O-acétyl-*α*-*D*-galactopyranosyle sont alors ajoutés et l'agitation est poursuivie pendant 12 h. Les

solides sent essorés et le filtrat est évaporé à sec. Le résidu est dissous dans 200 ml de chloroforme et la solution obtenue est lavée avec une solution aqueuse à 10% d'iodure de potassium, avec de l'eau, séchée ($CaCl_2$) et évaporée. Le résidu est dissous dans 100 ml de méthanol et 3 ml d'une solution molaire de méthylate de sodium dans le méthanol sont ajoutés. Au bout d'une heure, le milieu réactionnel est neutralisé (résine acide) et évaporé. Le résidu est cristallisé dans l'éthanol, donnant l'allyl-*β-D*-galactopyranoside (5,2 g, 81%); P.F. 103—104°C; $[\alpha]_D^{20}= -19°$ (*c* 1, MeOH);

*β*) allyl-4,6,O-benzylidène-*β*-D-galactopyranoside

On soumet ensuite 5,96 g d'allyl-*β-D*-galactopyranoside à une forte agitation pendant 12 h en présence de 80 ml d'aldéhyde benzoique et de 5,5 g de chlorure de zinc. Le milieu réactionnel est alors lentement versé sous forte agitation dans 300 ml de diisopropyléther. Le précipité formé est essoré au bout de quelques heures et lavé avec 100 ml d'éther environ. Le solide obtenu est cristallisé dans de l'éthanol, donnant l'allyl-4,6,O-benzylidène-*β-D*-galactopyranoside (6,92g, 82%); P.F. 178—179°C, $[\alpha]_D^{20}= -7°$ (*c* 1, pyridine); *anal.:* calc. pour $C_{16}H_{20}O_6$, $H_2O$: C: 58, 88, H: 6,79: 0:34, 31: trouvé: C; 58, 94; H: 6,47; O: 34,09. Ce produit a été caractérisé par un di-*O*-acétate: P.F. 94—95°C $[\alpha]_D^{20}= +56°$ (*c* 1, chloroforme) *anal.* cal. pour $C_{20}H_{24}O_8$: C: 61,22; H; 6,16; 0; 32,62; trouve: C: 61,31, H; 6 12; O: 32,77.

*δ*) allyl-3-O-benzoyl-4,6-O-benzylidène-*β-D*-galactopyranoside.

On ajoute à 5 ml d'une solution chloroformique anhydre renfermant 443 mg d'imidazole, une solution de 457 mg de chlorure de benzoyle dans 2 ml. de chloroforme anhydre à 5°C. Au bout de 10 mn, le chlorhydrate d'imidazole formé est essoré et lavé avec 5 ml de chloroforme. 20 ml d'une solution chloroformique anhydre d'allyl-4,6-O-benzylidène-*β-D*-galactopyranoside (1 g) est alors ajoutée au filtrat et le milieu réactionnel est porté à reflux à 75°C, pendant 18h environ. Après refroidissement à la température ambiante, le milieu réactionnel est dilué avec 50 ml de chloroforme, lavé avec un solution aqueuse diluée de bicarbonate, avec de l'eau, séché ($CaCl_2$) et évaporé. Le résidue est cristallisé dans le mélange acétate d'éthyle-éther, donnant le composé de formule XIV (1,14 g, 85%), P.F. 172—173°, $[\alpha]_D^{20}= +83°$ (*c* 1, chloroforme).

*anal.* calc. pour $C_{23}H_{24}O_7$: C: 66,98; H: 5,87; O: 27,16.

trouvé: C: 67,01; H: 5,75; O: 27,37.

c) Condensation des produits obtenus sous a) et b).

On soumet à agitation une solution de 619 mg du dérivé de formule XIV dans 20 ml de nitrométhane anhydre pendant 3 h à l'abri de l'humidité et sous azote sec en présence de tamis moléculaires 4 Å en poudre (1 g). On a joute à cette solution réactionnelle 1,222 g d'acétimidyl fucopyranose XIII et 344 mg d'acide *p*-toluènesulfonique et l'on poursuit l'agitation pendant 20 h. Le milieu réactionnel est alors neutralisé avec de la triéthylamine et filtré. Le filtrat est évapore et le résidu est purifié par chromatographie sur une colonne de 150 g de gel de silice à l'aide du mélange hexane-acétate d'ethyle (5:2, v/v). L'allyl-3-O-benzoyl-2-O-2,3,4-tri-O-benzyl-*α-L*-fucopyranosyl-4,6-O-benzylidène-*β*-galactopyranoside est obtenu à l'état pur (1,131 g, 91%), $[\alpha]_D^{20}= +13°$ (*c* 1, chloroforme), R.M.N. (chloroforme-*d*): *δ* 1,12(3H, d, 7 Hz, Me-C), 7,00—7,55 (20H, m, Ph).

*anal:* calc. pour $C_{50}H_{52}O_{11}$: C: 72,44; H: 6,32; O: 21,23..

trouvé: C: 72,66; H: 6,32; O: 21,27.

Ce disaccharide est soumis à une réaction de O-débenzoylation sélective en procédant comme suit: On dissout 1,03 g du dérivé de galactopyranoside en question dans 20 ml de méthanol et l'on ajoute 1 ml d'une solution méthanolique molaire de méthylate de sodium. Au bout d'une heure, le milieu réactionnel est concentré et versé dans de l'eau glacée. La phase obtenue est extraite avec du chloroforme, les extraits chloroformiques étant ensuite lavés avec de l'eau, séchés ($CaCl_2$) et évaporés. Le résidu est cristallisé dans le mélange acétate d'éthyleéther-hexane, donnant l'allyl 2-O-(2,3,4-tri-O-benzyl-*α-L*-fucopyrannosyl)-4,6-O-benzylidène-*β-D*-galactopyranoside (847 mg, 94%); P.F. 166—167°C, $[\alpha]_D^{20}= -58°$ (*c*, 1, chloroforme), spectre R.M.N. (chloroforme-*d*): *δ* 1,12 (3H, d, 7Hz, Me-C), 5,26 (1H, d, $J_{1',2'}$ 4Hz, H—1'), 7,15—7,65 (20H, M, Ph).

*anal.* calc. pour $C_{43}H_{48}O_{10}$: C: 71,52; H: 6,67; O: 22,07.

trouvé: C: 71,68; H: 6,63; O: 22,15.

2) Préparation du 1-O-(N-méthyl)-acétamidyl de formule XII

Cet imidate est préparé à partir du 2,3,4,6-tétra-O-benzyl-*D-D*-galactopyranose selon la méthode décrite sous 1-b). Le chlorure de 2,3,4,6-tétra-O-benzyl-*α-D*-galactopyranosyle formé intermédiarement est obtenu avec un rendement de 93%, $[\alpha]_D^{20}= +151°$ (*c* 2,5 benzène). L'imidate est obtenu avec un rendement de 92%, $[\alpha]_D^{20}= +25°$ (*c* 1, chloroforme), spectre R.M.N. (chloroforme-*d*): *δ* 1,84 (3H, s, Me-C), 2,95 (3H, s, Me-N), 5,83 (1H, d, $J_{1,2}$8 Hz, H-1), 730 (20H, m, pH).

*anal.* calc. pour $C_{37}H_{41}NO_6$: C: 74,60; H: 6,94; N: 2,35; O, 16,12.

trouvé: C: 74.68; H: 6,91; N: 2,10; O: 16,31.

3) Condensation des produits sous 1) et 2)

On soumet à agitation pendant 5 h, en présence de tamis moleculaires 4 Å (1 g) et sous atmosphère d'azote sec, une solution du composé allyl-2-O-(2,3,4-tri-O-benzyl-*α-L*-fucopyranosyl)-4,6,O-benzylidène-*β-D*-galactopyranoside (600 mg) et 900 mg de l'acétimidate de formule XII dans 15 ml de nitrométhane. On ajoute ensuite une solution d'acide *p*-toluènesulfonique anhydre (150 mg) dans 5 ml de nitrométhane. Le mélange réactionnel est soumis à agitation pendant 48 h à la température ambiante. Après addition de 1 ml de triéthylamine, le milieu réactionnel est filtré et le filtrat est évaporé à sec. Le résidu est dissous dans 50 ml de chloroforme, la solution étant lavée avec une solution aqueuse saturée de bicarbonate de sodium, avec de l'eau, séchée ($CaCl_2$) et évaporée. Le résidu est chromatographié sur une colonne de 80 g de gel de silice à l'aide du mélange hexane-acétate d'éthylle (5:2, v/v), conduisant au composé de formule X (907 mg. 88%) qui est cristallisé dans l'éthanol à 95% (865 mg, 84%), P.F. 117—118° $[\alpha]_D^{20}= +2°$ (*c*1, chloroforme), spectre R.M.N. (chloroforme-*d*): $\delta$ 1,18 (3H, d, 7 Hz, $CH_3$-C), 7,05—7,55 (40H, m, Ph)

*anal.* calc. pour $C_{77}H_{82}O_{15}$: C: 74,13; H: 6,62; O: 19,24.
trouvé: C: 74,22; H: 6,64; O: 19,54.

Exemple 2

Selon une variante, on prépare le produit de formule X en effectuant:

1/ La condensation de l'imidylgalactopyranose de formule XII avec l'allyl-galacetopyranose de formule XVI (l'allyl 2-O-benzoyl-4,6-O-benzylidène-*β-D*-galactopyranose):

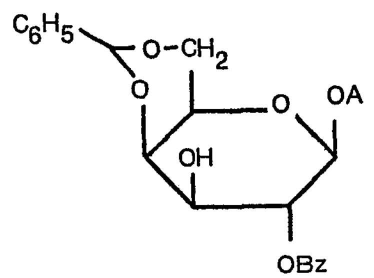

(XVI)

ce qui conduit au disaccharide allyl-2-O-benzoyl-3-O-(2,3,4,6-tétra-O-benzyl-*α-D*-galactopyranosyl)-4,6,O-benzylidène-*β-D*-galactopyranoside de formule:

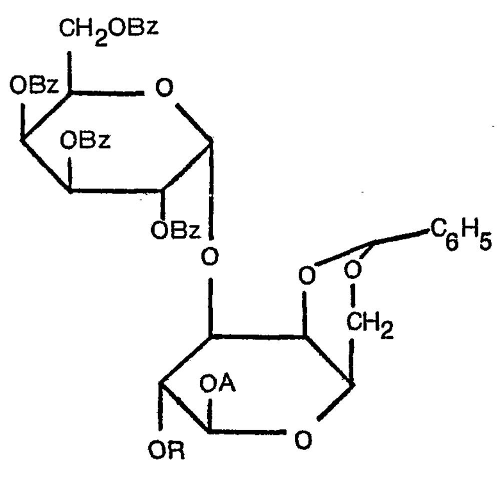

(XVII)

puis

2/La condensation du disaccharide de formule XVII préalablement O-débenzoylé, avec l'imidyl-fucopyrano se de formule XIII.

1) Condensation de l'imidyl-galactopyranose de formmule XII avec l'allylgalactopyranose de formule XVI

a) on prépare tout d'abord l'allyl-2-O-benzoyl-4,6-O-benzylidène-*β-D*-galactopyranoside en procédant comme suit: On ajoute 50 ml d'une solution glacée d'hydroxyde de soude à 15 ml d'une solution d'acétone renfermant 1,5 g du composé de formule XIV. On extrait au bout de deux minutes le milieu réactionnel avec 80 ml de chloroforme, les extraits organiques étant lavés avec de l'eau, séchés avec $CaCl_2$ et évaporés. Le résidu est soumis à une chromatographie sur une colonne de 80 g de gel de silice à l'aide du mélange acétate d'éthyle-hexane (1:1, v/v). On obtient ainsi le produit de départ

(782 mg, 52%) et le dérivé recherché (661 mg, 44%), P.F. 144—145°C, $[\alpha]_D^{20}$= +10,2° (*c* 1, chloroforme).
*anal.* calc. pour $C_{23}H_{24}O_7$: C: 66,98; H: 5,87; O: 27,16.
trouvé: C: 67,04; H: 6,03; O: 27,12.

b) on effectue la galactosylation du galactopyranoside ainsi formé à l'aide du composé de formule XII. On opère selon la technique précédemment décrite en mettant en oeuvre 285 mg du galactopyranoside et 596 mg de l'imidate. Après purification sur une colonne de gel de silice (établie avec 40 g de silice et une mélange acétate d'éthyle-hexane 2: 1 v/v), on obtient 594 mg du disaccharide recherché, ce qui correspond à un rendement de 95%. $[\alpha]_D^{20}$= +80,5° (*c* 1, chloroforme), spectre R.M.N. (chloroforme-*d*): $\delta$ 4,63 (1H, d, $J_{1,2}$ $\gamma$ Hz, H-1), 5,13 (1H, d, $J_{1',2'}$ 4Hz, H-1').
*anal.* calc. pour $C_{57}H_{58}O_{12}$: C: 73,21; H: 6,25; O: 20,53.
trouvé: C: 73,06; H: 6,02; O: 20,71.

c) le disaccharide obtenu est soumis à une O-débenzoylation sélective. On dissous 580 mg du disaccharide dans 20 ml de méthanol, puis on le traite par 1 ml d'une solution méthanolique 1 M de méthylate de sodium. 12 heures après, on concentre le milieu réactionnel, dilué avec 50 ml de chloroforme, puis on le lave avec de l'eau, on le séche avec $CaCl_2$ et on évapore. Le résidu est cristallisé dans le méthanol à 95°. On obtient 479 mg, rendement 93% du disaccharide O-débenzoylé. P.F. 150—151°C, $[\alpha]_D^{20}$= +44° (*c* 1,chloroforme), spectre R.M.N. (chloroforme-*d*): $\delta$ 2,86 (1H, d, J 3 Hz, OH), 5,21 (1H, d, $J_{1',2'}$ 4Hz, H-1'), 7,10—7,60 (25H, m, Ph),
*anal.* calc. pour $C_{50}H_{54}O_{11}$: C: 72,27; H: 6,55; O: 21,18.
trouvé: C: 72,46; H: 6,52; O: 21,29.

d) Selon la technique décrite ci-dessus, on prépare le trisaccharide de formule X en condensant le disaccharide de formule XVII avec l'imidate cristallin de formule XIII, mis en oeuvre respectivement à raison de 275 et 325 mg. On obtient 370 mg de trisaccharide, ce qui correspond à un rendement de 79%. P.F. 115—116°C, $[\alpha]_D^{20}$+2° (*c*1, chloroforme.)

## Exemple 3

Preparation du 2 - O - (2,3,4 - tri - O - benzyl - *α* - *L* - fucopyranosyl) - 3 - O - (2,3,4,6 - tétra - O - benzyl - *α* - *D* - galactopyranosyl) - 4,6 - O - benzylidène - *D* - galactopyranose, de formule:

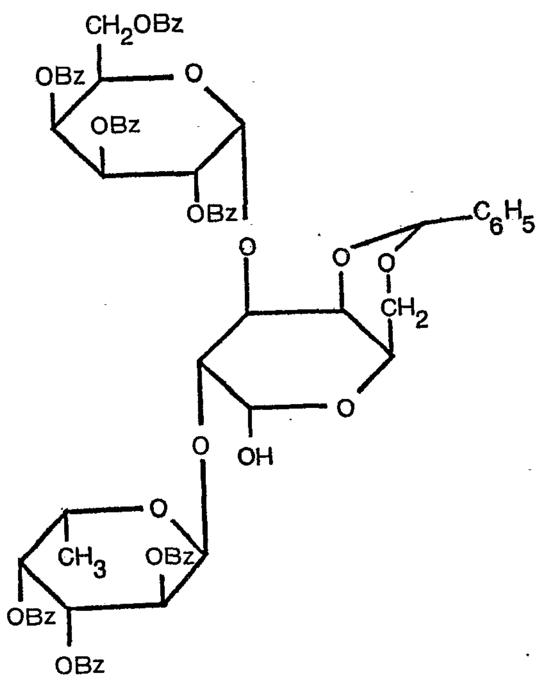

(XIX)

On dissout 624 mg de trisaccharide de formule X dans 10 ml de diméthylsulfoxyde. Le mélange réactionnel ainsi formé est soumis à agitation pendant environ 4 h à 100°C, en présence de 294 mg de tert-butoxyde de potassium. Après refroidissement à la température ambiante, on verse le milieu réactionnel dans une solution aqueous glacée à 10% de chlorure d'ammonium; cette solution est extraite avec 60 ml de chloroforme, les extraits chloroformiques étant lavés de l'eau, séchés avec $CaCl_2$ et évaporés. On dissout le résidu dans 20 ml d'un mélange acétone-eau (9:1,v/v), puis on soumet la

solution à agitation pen-dans 30 mn en présence de 428 mg d'oxyde mercurique jaune et de 271 mg de chlorure mercurique. Après filtration, on lave le milieu réactionnel avec une solution aqueuse à 10% d'iodure de potassium, avec de l'eau, on le séche avec $CaCl_2$ puis on évapore. Le résidu est soumis à une chromatographie sur une colonne de gel de silice (50 g) à l'aide du mélange hexane-acétate d'éthyle (3:2,v/v). On obtient ainsi 495 mg du trisaccharide recherché (rendement 82%) sous forme d'une mousse incolore $[\alpha]_D^{20}$+50° (*c* 1, méthanol), spectre R.M.N. (chloroforme-*d*): $\delta$ 2,99 (3H, d, 7 Hz, $CH_3$-C), 5,56 (1H, s, CH Ph), 7,05—7,65 (40H, m, Ph).
*anal.* calc. pour $C_{74}H_{78}O_{15}$: C: 73:61; H: 6,51; O: 19,87.
trouvé: C: 73,80; H: 6,58; O: 20,06.

Exemple 4

Préparation du trisaccharide B ou 2-O-($\alpha$-*L*-fucopyranosyl)-3-O-($\alpha$-*D*-galactopyranosyl)-*D*-galactopyranose de formule:

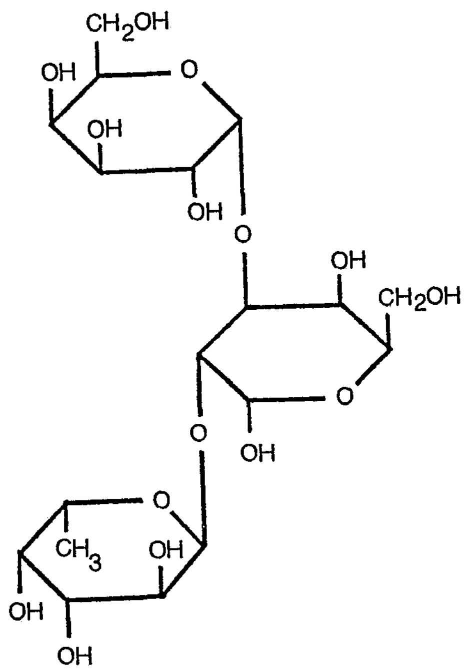

(I)

On soumet 480 mg du trisaccharide de formule XIX à une hydrogénolyse pendant environ 20 h à la température ambiante et à la pression atmosphérique dans 20 ml d'acide acétique en présence de 500 mg de palladium sur charbon. Après essorage du catalyseur, le filtrat est évaporé à sec à une température inférieure à 35°C, donnant le trisaccharide recherché (186 mg, 96%) sous la forme d'une poudre planche. P.F. 135—138°C (décomposition), $[\alpha]_D^{20}$+35° (*c*1, eau: méthanol, 19:1, v/v), spectre R.M.N. ($D_2O$): $\delta$ 1,70 (3H, d, 7 Hz, $CH_3$-C). Ce composé est caractérisé par un peracétate cristallin, P.F. 215—216°C $[\alpha]_D^{20}$= +51° (c 1, chloroforme) spectre R.M.N. (chloroforme-*d*): $\delta$ 1,17 (3H, d, 6,5 Hz, Me-C), 1,90—2,20 (30H, m, AC), 6,40 (1H, d, $J_{1,2}$3,5 Hz, H-1, anomère $\alpha$).

Exemple 5

Preparation du $\beta$ - hydroxy - éthyl - 2 - O - (2,3,4 - tri - O - benzyl - $\alpha$ - *L* - fucopyranosyl) - 3 - O - (2,3,4,6 - tetra - O - benzyl - $\alpha$ - *D* - galactopyranosyl) - 4,6 - O - benzylidène - $\beta$ - *D* - galactopyranoside, de formule XX

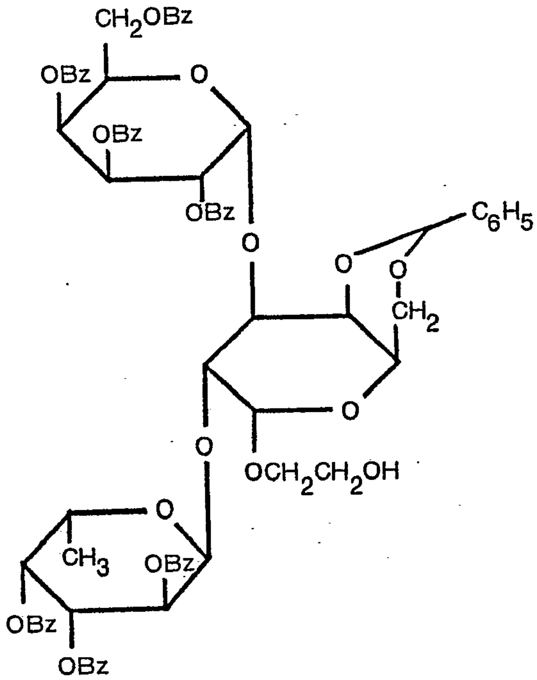

(XX)

On agite à température ambiante 312 mg de trisaccharide X (0,25 mole) dans 16 ml d'un mélange dioxanne-eau (3:1, v/v) auquel on ajoute 1,25 mg de tétroxyde d'osmium ($OsO_4$). Au bout d'une heure, on ajoute 112 mg de periodate de sodium ($NaIO_4$), 2,1 éq., puis on agite 4 h environ. On ajoute 50 mg de borhydrure de sodium ($NaBH_4$) puis, après 10 mn, on évapore à sec et on reprend le résidu dans 100 ml environ de chloroforme. Cette solution chloroformique est lavée avec une solution aqueuse de sulfite de sodium ($Na_2SO_3$) à 10%, puis avec de l'eau. On sèche ensuite sur du chlorure de calcium ($CaCl_2$) puis on filtre et on évapore le filtrat. On obtient un sirop qui est traité avec 16 ml d'un mélange dioxanne-eau (3:1 v/v) et soumis à agitation evec 52 mg de $NaIO_4$ (1 éq.). Au bout de 3 h, on ajoute 50 mg de $NaBH_4$, puis 10 mn après, on évapore à sec, et l'on reprend le résidu dans 100 ml de chloroforme. On procède au lavage de la solution chloroformique comme indiqué ci-dessus. Le sirop obtenu est soumis à une chromatographie sur 20 g de silice ($SiO_2$). On utilise comme éluant un mélange d'acétate d'éthyle et d'hexane (3:1 v/v). Le produit recueilli aprè évaporation dans l'éluant est cristallisé dans un mélange éther-hexane. On obtient 247 mg du trisaccharide recherché; P.F. 83—84°C rendement 79%.

Exemple 6

Préparation du $\alpha$ - hydroxypropyl - 2 - O - (2,3,4 - tri - O - benzyl - $\alpha$ - L - fucopyranosyl) - 3 - O - (2,3,4,6 - tetra - O - benzyl - $\alpha$ - D - galactopyranosyl) - 4,6 - O - benzylidène - $\beta$ - D - galactopyranoside, formule XXI:

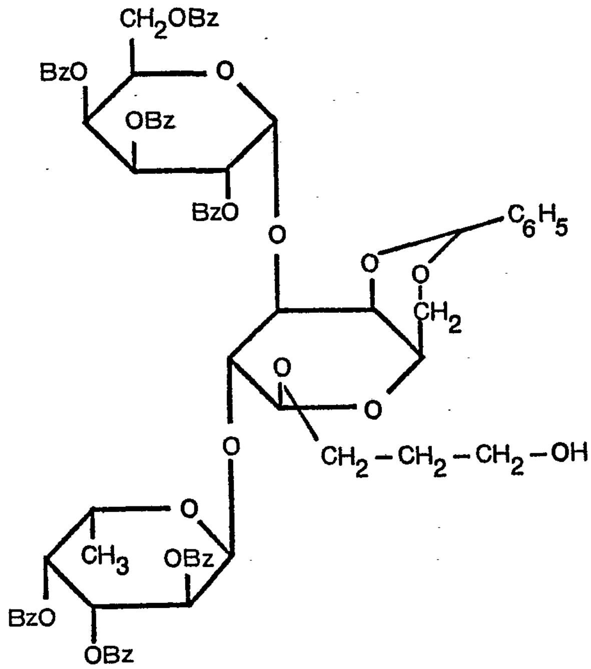

(XXI)

On agite à température ambiante 312 mg du trisaccharide X (0,25 mmole) dans du tétrahydrofuranne anhydre (3 ml) et on ajoute 0,8 ml d'une solution 0,5 M de 9-borabicyclo[3.3.1] nonane ou 9—BBN dans le tetrahydrofuranne. Après 4 h de chauffage à reflux et refroidissement à la température ambiante, l'excès de 9—BBN est détruit par addition d'éthanol (0,3 ml). On ajoute ensuite une solution aqueuse 3 M d'hydroxyde de sodium (0,2 ml), puis, goutte à goutte, 0,2 ml d'une solution 10 m d'eau oxygénée, en maintenant la température entre 40 et 50°C. Le melange réactionnel est ensuite chauffé à 50°C sous agitation pendant 1h. Après refroidissement, du carbonate depotassium solide (1g) et du dichlorométhane (20 ml) sont ajoutés. Les solides sont essorés et le filtrat est évaporé. Le résidu est purifié par passage sur une colonne de gel de silice (15 g) et élution à l'aide du mélange acétate d'éthylehexane (2:1, v/v). Le composé XXI est ainsi obtenu à l'état pur (292 mg, 92%), $[\alpha]_D^{20}$ +2° (*c* 1, chloroforme). Ce composé est caracterisé par un acétate cristallin, P.F. 1128113°C, $[\alpha]_D^{20}$ +1,5° (*c* 1, chloroforme), spectre R.M.N. (chloroforme-*d*): $\delta$ 1,20 (3H, d, 6, 5 Hz, Me-C), 2,00 (s, 3H, Ac), 7,10—7,60 (40H, m, Ph).

Exemple 7

Préparation du $\gamma$-allyloxypropyl - 2 - O - (2 - O - (2,3,4 - tri - *O* - benzyl - $\alpha$ - *L* - fucopyranosyl) - 3 - *O* - (2,3,4,6 - tetra - *O* - benzyl - $\alpha$ - *D* - galactopyranosyl) - 4,6 - *O* - benzylidène - $\beta$ - *D* - galactopyranoside, de formule XXII:

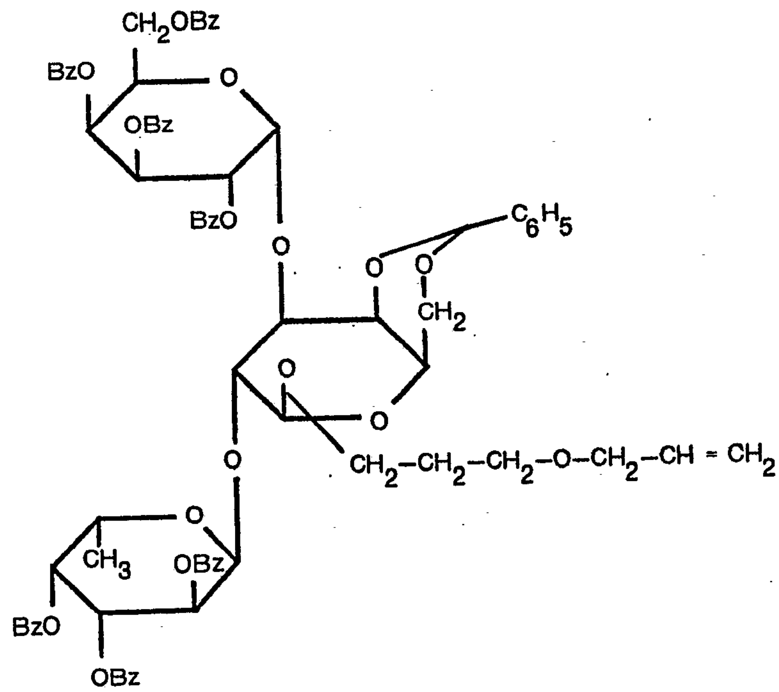

(XXII)

On agite à température ambiante 633 mg du composé XXI dans du *N,N*-diméthylformamide (10 ml) en présence d'hydrure de sodium (96 mg de dispersion à 50% dans de l'huile). Au bout d'une heure, du bromure d'allyle (0,15 ml) est ajouté. La réaction est complète en 1h30. Après addition de méthanol (2 ml), le milieu réactionnel est évaporé et le résidu repris dans du chloroforme (50 ml). La phase organique est lavée avec une solution aqueuse saturée de chlorure de sodium, avec de l'eau, séchée sur sulfate desodium, filtrée et évaporée. L'huile contenue dans le résidu est extraite avec de l'hexane bouillant (20 ml), le composé cherché est cristallisé dans un mélange acétate d'éthyle-hexane (588 mg, 90%), P.F. 103—104°C, $[\alpha]_D^{20}$ +3° (*c* 1, chloroforme), spectre R.M.N. (chloroforme-*d*): $\delta$ 1,21 (3H, d, J 7Hz, Me-C), 7,10—7,55 (40H, m, Ph).

Exemple 8

Préparation du $\gamma$[($\gamma$ - hydroxy) propyloxy]propyl - 2 - *O* - (2,3,4 - tri - *O* - benzyl - $\alpha$ - *L* - fucopyranosyl) - 3 - *O* - (2,3,4,6-tétra - *O* - benzyl - $\alpha$ - *D* - galactopyranosyl) - 4,6 - *O* - benzylidène - $\beta$ - *D* - galactopyranoside, de formule XXIII:

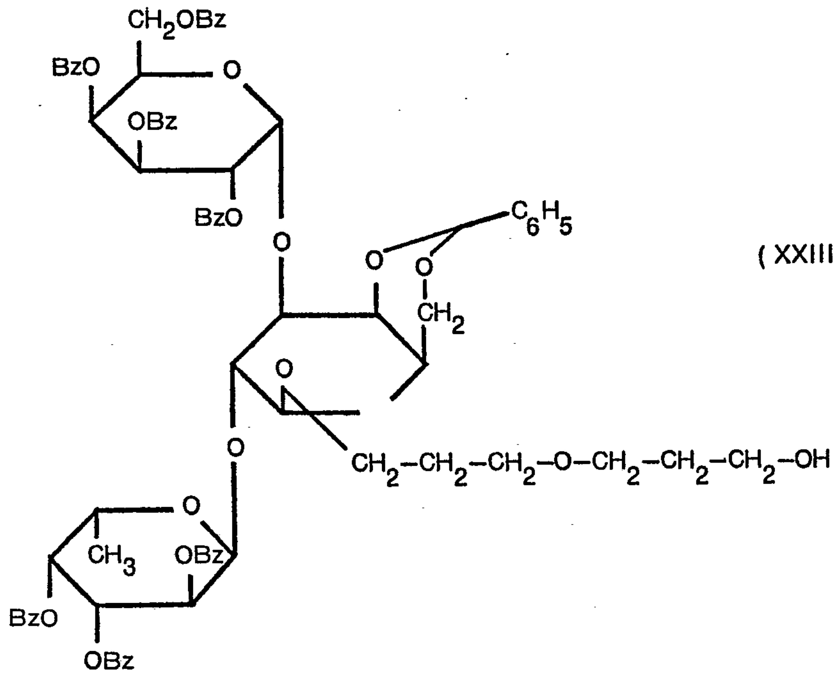

(XXIII)

On agite à température ambiante 490 mg du dérivé XXII dans du tétrahydrofuranne anhydre (4 ml) et on ajoute 1 ml d'une solution 0,5 M de 9—BBN dans le tétrahydrofuranne. Après 2 h30 de chauffage à reflux refoidissement à la température ambiante, l'excès de 9—BBN est détruit par addition d'ethanol (1 ml). On ajoute ensuite une solution aqueuse 3 M d'hydroxyde de sodium (0,25 ml) puis, goutte à goutte, 0,38 ml d'une solution 10 M d'eau oxygénée, en maintenant la température entre 40 et 50°C. Le mélange réactionnel est ensuite chauffé à 50°C pendant 1 h. Après refroidissement, la phase aqueuse est saturée avec du carbonate depotassium (1 g), filtrée et évaporée. Le résidu est purifié par passage sur une colonne de gel de silice (30 mg) et élution à l'aide du mélange acétate d'éthyle-hexane (2:1, v/v). Le composé XXIII est ainsi obtenu à l'état pur (442 mg, 89%), $[\alpha]_D^{20}$ +1,5° (*c* 1, chloroforme), spectre R.M.N. (chloroforme-*d*): $\delta$ 1,20 (3H, d, 6 5Hz, Me—C), 7,60—7,70 (40H, m, Ph).

## Exemple 9

Préparation du composé de formule XXIV:

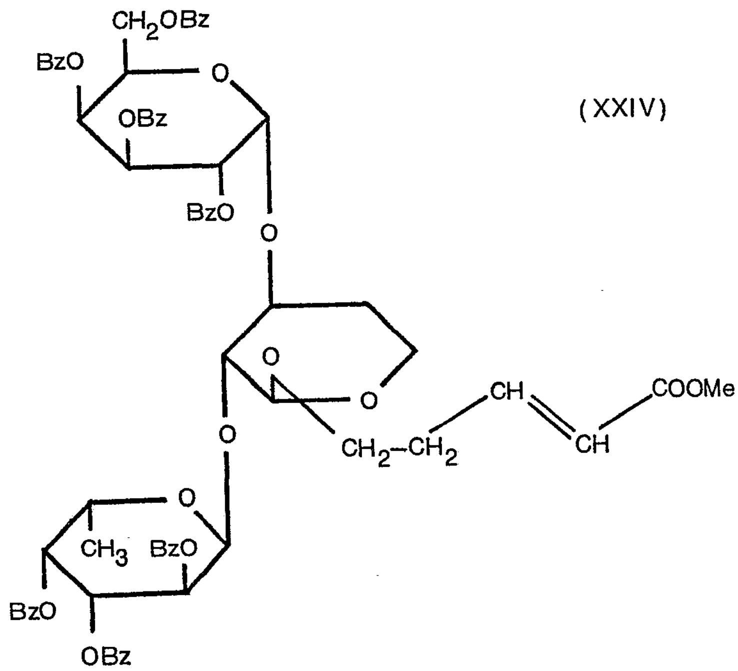

(XXIV)

A un mélange de dichlorométhane (15 ml) et de pyridine (0,644 ml) ou ajoute, sous agitation et à l'abri de l'humidité, du trioxyde de chrome sec (400 mg) Après 20 minutes d'agitation, une solution du trisaccharide XXI (633 mg) dans le dichlorométhane (5 ml) est ajoutée. Au bout de 20 minutes, le milieu réactionnel est décanté, le résidu étant extrait au chloroforme. La phase chloroformique est lavée avec une solution saturée de bicarbonate de sodium, avec de l'eau, séchée sur sulfate de sodium, filtrée et évaporée. Le résidu est purifié par chromatographiesur gel de silice (10 g) à l'aide du mélange acétate d'éthyle-hexane (3:1, v/v). L'aldéhyde obtenu (575 mg, 91%) est transformé en dérivé XXIV grâce à une réaction de Wittig classique dans le benzène (15 ml). Ce dérivé est cristallisé dans éther-hexane (524 mg, 88%), P.F. 152—153°C, $[\alpha]_D^{20}$ −3° (*c* 1, chloroforme), spectre R.M.N. (chloroforme-*d*): $\delta$ 1,23 (3H, d, 6,5 Hz, Me-C), 3,69 (3H, s, COOMe), 7,10—7,55 (40H, m, Ph).

## Exemple 10

Application de trisaccharides de l'invention en tant qu'immunoabsorbants:

*a*) On procède à la fixation du trisaccharide XXIV sur des supports à base d'agarose.

1°) On utilise une matrice d'agarose activée recouverte par de la sérum albumine humaine. L'activation de l'agarose est réalisée avec du bromure de cyanogène CNBr selon la méthode de Axen et al décrite dans Nature, *214*, 1302 (1967) modifiée par March et al suivant la technique décrite dans Anal. Biochem, *60* 149 (1964). Pour recouvrir l'agarose par de la sérum albumine humaine, on opère suivant la méthose de Parikh et al décrite dans "Methods in enzymology", *35*, 77 (1974)—1 ml. de gel d'agarose tassé contient environ 2 mg d'albumine. On effectue le couplage du trisaccharide XXIV sur le dérivé d'agarose-albumine à l'aide de 1-cyclohexyl-3(2-morpholinoéthyl) carbodiimide-métho-p-toluène-sulfonate en opérant selon les techniques connues dans ce domaine, en particulier celle de Parikh et al dont question ci-dessus, ou de Weetall et al dans le même ouvrage, p. 59, ou encore de Hoare et al dans JACS *88,* 2057, (1966) ou J. Biol. Chem. *242,* 2447 (1967). On introduit ainsi environ 10 à 20 molécules de trisachhariode XXIV par molécule d'albumine.

2°)—Dans une variante, le support est constitué par de l'agarose activée indiqué ci-dessus, transformée ensuite en un dérivé d'aminoalcoylagarose à l'aide d'ethylènediamine selon le mode opératoire décrit par Cohen dans Methods in enzymology *35,* 102 (1974). Le couplage du dérivé d'agarose obtenu avec le trisaccharide XXIV est effectué à l'aide du dérivé de carbondiimide comme exposé plus haut. On procède ensuite à l'acétylation du produit de couplage pour éliminer l'excès de groupements amino.

*b*)—On constitue une autre série d'immunoabsorbants en fixant un dérivé de trisaccharide de formule II dans laquelle *R* représente un atome d'hydrogène et *A* est un groupe $-(CH_2)_3-O-(CH_2)_4NH_2$ sur de la carboxy-méthyl cellulose. Le couplage est effectué à l'aide du dérivé de carbodiimide comme décrit plus haut.

Exemple 11

Utilisation du trisaccharide de formule XXIV pour la neutralisation des hémolysines anti-B. On utilise une solution à 10 mg/ml du produit de formule XXIV. En opérant selon les techniques de la pharmacopée européenne, on constate une inhibition totale d'une dose agglutinante d'un sérum-test anti-B avec une dilution 1/5 du trisaccharide de l'invention.

**Revendications**

1. Procédé de préparation de dérivés d'osides, caractérisé par le fait qu'on fait réagir:
—un dérivé d'oside a) constitué par un ou plusieurs motifs oses, éventuellement rattaché (s) à un reste organique, ce motif ose, ou l'un au moins de ces motifs oses, étant substitué, sur le carbone anomère en position 1 par un groupe —O— imidyle de formule —O—C(—$R_2$)=N—$R_1$ dans lequel les substituants $R_1$ et $R_2$, identiques ou différents l'un de l'autre, représentent un radical alcoyle comprenant, de préférence, 1 à 4 atomes de carbone, les groupes —CH du dérivé d'oside étant protégés par des groupes appropriés, avec
—un dérive d'oside b) constitué par un ou plusieurs motifs oses, éventuellement rattaché (s) à un reste organique, un seul groupe —OH de ce ou ces motifs oses étant libre et occupant l'une quelconque des positions hydroxyle secondaire 1 à 4, les dérivés d'osides a) et/ou b) comprenant un motif avec au moins deux oses.

2. Procédé de préparation de dérivés d'osides, caractérisé par le fait que, pour obtenir des osides branchés, on fait réagir le dérivé d'oside a) selon la revendication 1 avec un dérivé d'oside constitué par un ou plusieurs motifs oses, éventuellement rattaché (s) à un reste organique, un seul groupe —OH de ce ou ces motifs étant libre et occupant une position hydroxyle 1 à 4 ou 6 permettant la formation d'une chaîne osidique branchée.

3. Procédé selon la revendication 1 ou 2, caractérisé par le fait que le dérivé d'oside a) mis en oeuvre est constitué par un motif galactopyranose.

4. Procédé selon la revendication 3, caractérisé par le fait que le dérivé d'oside a) est constitué par un motif fucopyranose.

5. Procédé selon l'une quelconque des revendications 1 a 4, caractérisé par le fait que le dérivé d'oside b) comprend un motif ose comportant en position 1 une substitution —OA constituée par un groupe fonctionnel, non réactif dans les conditions dela synthèse osidique ou rendu non réactif, ou par un groupe fonctionnalisable, c'est-à-dire autorisant au cours ou en fin de synthèse l'introduction de groupements fonctionnels.

6. Procédé selon la revendication 5, caractérisé par le fait que, dans la substitution —OA, *A* représente un radical comportant une ou plusieurs liaisons insaturées, tel qu'un radical éthyléniquement insaturé, de preférence le radical alcényle comprenant de 2 a' 10 atomes de carbone, ainsi que tout radical auquel peut donner lieu, selon les techniques classiques de synthèse organique, la fonctionnalisation de la ou des liaisons insaturées en question.

7. Procédé selon la revendication 5, caractérisé par le fait que *A* représente un radical alcoyle comportant de 2 à 10 atomes de carbone, et substitué par au moins un groupe —OH qui, le cas échéant, est protégé par un groupe de blocage ou engagé dans un radical fonctionnel, ou le radical allyle, ou un groupe fonctionnel renfermant au moins un radical éther et/ou amine.

8. Procédé de préparation d'un dérivé de trisaccharide branché comprenant un enchaînement galactopyranose-galactopyranose-fucopyranose et répondant à la structure II:

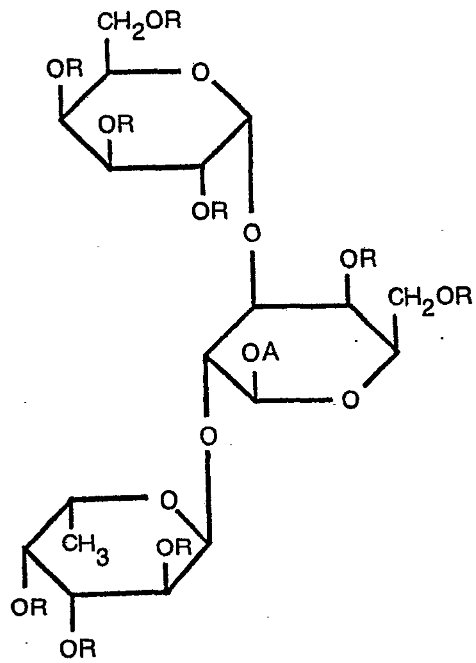

(II)

caractérisé par le fait qu'on fait réagir
—un 1-0-imidyl-*β-D*-galactopyranose de formule III:

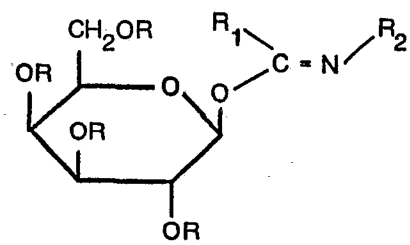

(III)

avec un disaccharide constitué par un fuco-galactopyranose comportant un groupement —OH libre en position 3 du motif galactopyranose, ce disaccharide répondant à la formule IV:

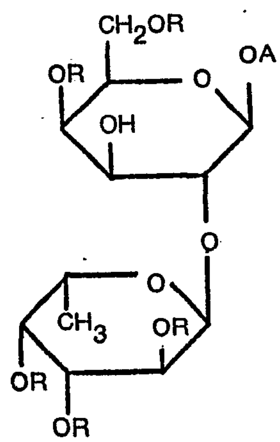

(IV)

dans lequelles:
—les substituants *R,* identiques ou différents les uns des autres, représentent des groupes de protection de radicaux hydroxyle, le cas échéant avec un substituant voisin, et sont choisis parmi des groupes stables, non réactifs dans les conditions habituelles de synthèse osidiques et facilement éliminables, dans des conditions douces compatibles avec le maintien de la structure osidique, en

particulier par des groupes constituant avec l'atome d'oxygène de l'hydroxyle des éthers benzyliques ou des acétals benzylidéniques,

—*A* représente un reste organique tel que défini dans l'une quelconque des revendications 6 et 7, et

—$R_1$ et $R_2$ présentent les significations données dans la revendication 3.

9. Procédé de préparation du dérivé de trisaccharide de formule II, selon la revendication 8, caractérisé par le fait qu'on met en oeuvre un digalactopyranose, comportant un groupement —OH libre en position 2 de l'un des motifs galactopyranose, et répondant à la formule V:

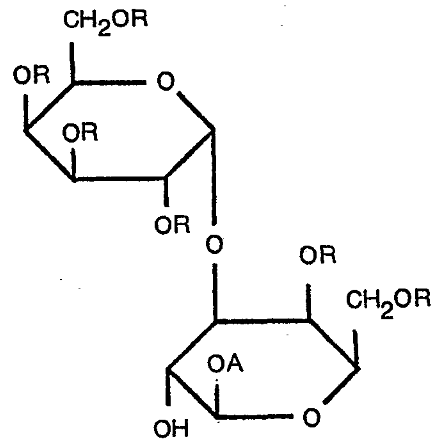

(V)

et qu'on effectue sa condensation avec le groupe -O-imidyle d'un 1-O-imidyl-*β*-*L*-fucopyranose de formule VI:

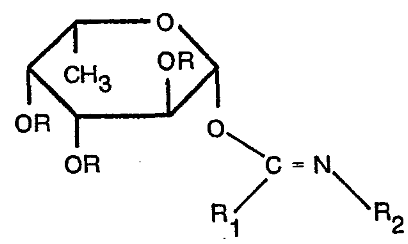

(VI)

$R_1$, $R_2$, *R* et *A* présentent dans ces formules les significations rappelées dans la revendication.

10. Procédé selon la revendication 8 ou 9, caractérisé par le fait que le substituant *A* du disaccharide de formule IV est constitué par le radical allyle.

11. Procédé selon l'une quelconque des revendications précédentes, caractérisé par le fait qu'on opére en présence d'acide p-toluène sulfonique anhydre et de nitrométhane.

12. Application du procédé selon l'une quelconque des revendications 8 à 11, à la synthèse du trisaccharide B, caractérisée par le fait qu'on procède à l'élimination séquentielle, dans des conditions douces selon les techniques classiques, du substituant *A* du produit de structure II obtenu, ce qui conduit à un produit de structure IX:

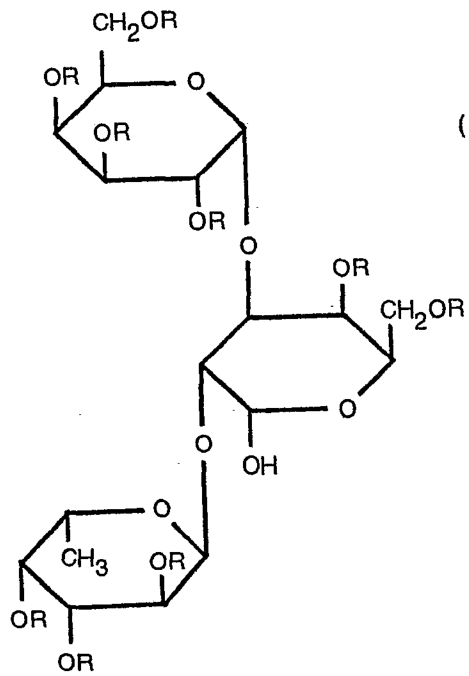

(IX)

suivie d'une hydrogénolyse.

13. Nouveaux osides tels qu'obtenus par réaction

—d'un dérivé d'oside a) constitué par un ou plusieurs motifs oses, éventuellement rattaché (s) à un reste organique, ce motif ose, ou l'un au moins de ces motifs oses, étant substitué, sur le carbone anomère en position 1, par un groupe —O— imidyle de formule $—O—C(—R_2)=N—R_1$ dans laquelle les substituants $R_1$ et $R_2$, identiques ou différents l'un de l'autre, représentent un radical aryle ou un radical alcoyle comprenant de préférence 1 à 4 atomes de carbone, les groupes —OH du dérivé d'oside étant protégés par des groupes appropriés, avec

—un derivé d'oside (b) constitué par un ou plusieurs motifs oses, éventuellement rattaché (s) à un reste organique, un seul groupe —OH de ce ou ces motifs oses étant libre et occupant l'une quelconque des positions hydroxyle secondaire, 1 à 4, les dérivés d'osides a) et/ou b) comprenant un motif avec au moins deux oses, ces osides étant caractérisé en ce qu'ils comportent au moins un motif galactopyranose dans lequel l'atome d'hydrogène du groupe —OH en position 1 est substitué par un radical *A* représentant un groupe alcoyle substitué par un moins un groupe hydroxyle, le cas échéant protégé par un groupe de blocage ou engagé dans un radical fontionnel, ou encore un groupe comportant une ou plusieurs liaisons insaturées, en particulier un radical éthyléniquement insaturé choisi parmi les radicaux alcényle, et plus spécialement les radicaux alcényle à 2 à 10 atomes de carbone, ou un radical alcoxyl-alcényle, alcoxy-alcool, alcoxy-ester, -amide ou -amine, alcoxy-alcoyl-amine ou alcoyle-amide ou alcoxy-acide carboxylique, ces différents groupes chimiques pouvant comporter en outre une fonction amine intercalaire.

14. Nouveaux osides selon la revendication 13, caractérisés par le fait que le radical *A* représente un radical alcoyle, comportant de 2 à 10 atomes de carbone, et substitué par au moins un groupe —OH qui, le cas échéant, est protégé par un groupe de blocage ou engagé dans un radical fonctionnel.

15. Nouveaux osides selon la revendication 13, caractérisés par le fait que *A* représente le radical allyle.

16. Nouveaux osides selon la revendication 13, caractérisés par le fait que *A* représente un groupe fonctionnel renfermant au moins un radical ether et/ou amine.

17. Nouveaux osides selon l'une quelconque des revendications précédentes, caractérisés par le fait que ledit motif galactopyranose est impliqué dans une chaîne trisaccharidique linéaire ou branchée comprenant un motif fucopyranose et un autre motif galactopyranose.

18. Nouveaux osides selon la revendication 17, caractérisés par le fait qu'il s'agit de trisaccharides branchés de formule II

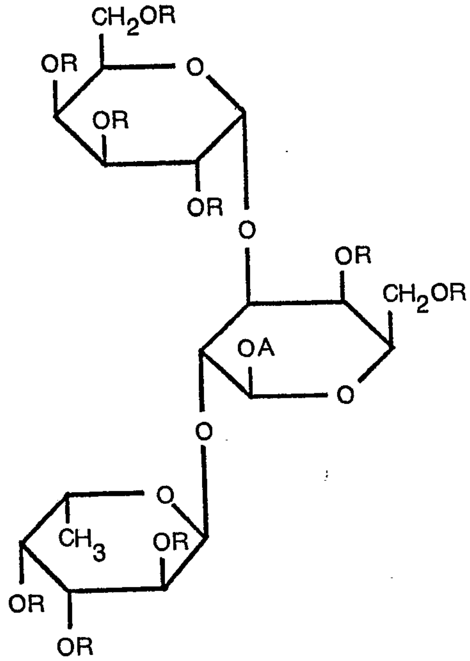

(II)

dans laquelle:

—les substituants *R*, identiques ou différents les uns des autres, représentent, le cas échéant avec un substituant voisin, des groupes de protection de radicaux hydroxyle et sont choisis parmi des groupes stables, non réactifs dans les conditions habituelles de synthèse osidique et facilement éliminables, dans des conditions douces compatibles avec le maintien de la structure osidique, en particulier par des groupes benzyle ou benzylidényle, ou représentent un atome d'hydrogène,

—*A* représente un reste organique comportant au moins un groupe fonctionnalisable constitué en particulier par un radical éthyléniquement insaturé choisi parmi les radicaux alcényle, de préférence de 2 à 10 atomes de carbone, et plus particulièrement constitué par le radical allyle, ou encore par un radical hydroxyalcoyle, plus spécialement $\beta$-hydroxyalkoyle, $\gamma$-hydroxypropyle, $\gamma$-allyloxypropyle, $\gamma[(\gamma$-hydroxy)propyloxy]propyle, le groupe $—CH_2—CH_2—CH{=}CH—COOCH_3$ ou encore le $—(CH_2)_3—O—(CH_2)_4NH_2$, le ou les groupes hydroxyle de ces radicaux étant éventuellement progégés ou bloqués.

19. Les trisaccharides selon la revendication 18, dans les quels les différents substituants *R* représentent un atome d'hydrogène.

20. Les trisaccharides de formule II selon la revendication 18, dans laquelle A représente un groupement allyle.

21. Application des trisaccharides selon la revendication 20 à la synthése du trisaccharide B selon le procédé de la revendication 12.

22. Applications biologiques des osides, selon l'une quelconque des revendications 13 à 20, comme immunoabsorbants ou pour la constitution d'un antigène artificiel.

23. Applications des osides selon l'une quelconque des revendications 13 à 20, sous forme libre ou immobilisés sur support à la constitution de réactifs biologiques, en particulier, pour la détection et la neutralisation des hémolysines.

24. Applications des osides selon l'une quelconque des revendications 18 à 20, sous forme libre ou immobilisés sur support, à la préparation de sérum-tests présentant une spécificité donnée.

**Claims**

1. Process for the preparation of oside derivatives, characterised in that an oside derivative a) which consists of one or more ose units optionally attached to an organic radical, this ose unit, or at least one of these ose units, being substituted, on the anomeric carbon in the 1-position, by a group —O— imidyl of the formula $—O—C(—R_2){=}N—R_1$, in which the substituents $R_1$ and $R_2$, which are identical or different from one another, represent an alkyl radical preferably containing 1 to 4 carbon atoms, and the groups —OH of the oside derivative being protected by suitable groups, is reacted with an oside derivative b) which consists of one or more ose units optionally attached to an organic radical, only one group —OH of this or these ose units being free and occupying any one of the secondary

hydroxyl positions 1 to 4, the osides derivatives a) and/or b) comprising one unit having at least two oses.

2. Process for the preparation of oside derivatives, characterised in that, in order to obtain branched osides, the oside derivative a), according to Claim 1, is reacted with an oside derivative which consists of one or more ose units optionally attached to an organic radical, only one group —OH of this or these units being free and occupying a hydroxyl position 1 to 4 or 6, this permitting the formation of a branched oside chain.

3. Process according to Claim 1 or 2, characterised in that the oside derivative a) employed consists of a galactopyranose unit.

4. Process according to Claim 3, characterised in that the oside derivative a) consists of a fucopyranose unit.

5. Process according to any one of Claims 1 to 4, characterised in that the oside derivative b) comprises an ose unit containing, in the 1-position, a substituent —OA consisting of a functional group which is unreactive under the conditions of the oside synthesis or has been rendered unreactive, or consisting of a group into which functional groups can be introduced, that is to say a group which permits the introduction of functional groups during or at the end of the synthesis.

6. Process according to Claim 5, characterised in that, in the substituent —OA, A represents a radical containing one or more unsaturated bonds, such as an ethylenically unsaturated radical, preferably an alkenyl radical containing from 2 to 10 carbon atoms, and also represents any radical which can be produced, in accordance with the conventional techniques of organic synthesis, by the introduction of functional groups into the unsaturated bond or bonds in question.

7. Process according to Claim 5, characterised in that, *A* represents an alkyl radical which contains. 2 to 10 carbon atoms and is substituted by at least one OH— group which, if necessary, is protected by a blocking group or forms part of a functional radical, or the allyl radical, or a functional group containing at least one ether and/or amide radical.

8. Process for the preparation of a branched trisaccharide derivative which comprises a galactopyranose-galactopyranose-fucopyranose linkage and corresponds to the structure II:

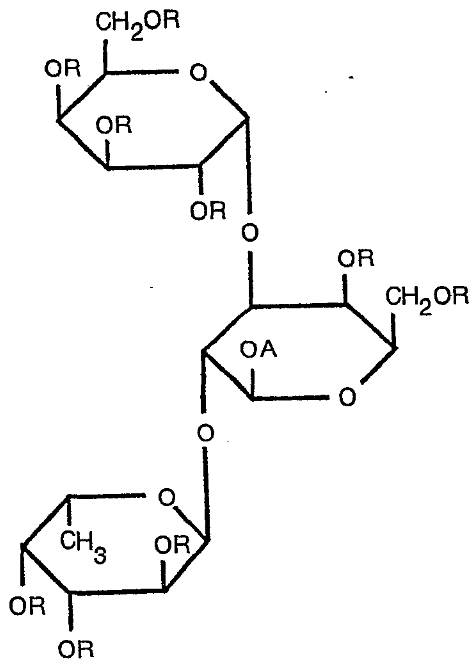

(II)

characterised in that a 1-O-imidyl-$\beta$-*D*-galactopyranose of the formula III:

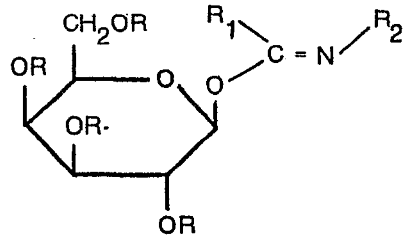

(III)

is reacted with a disaccharide consisting of a fuco-galactopyranose containing a free group —OH in the

3-position of the galactopyranose unit, this disaccharide corresponding to the formula IV:

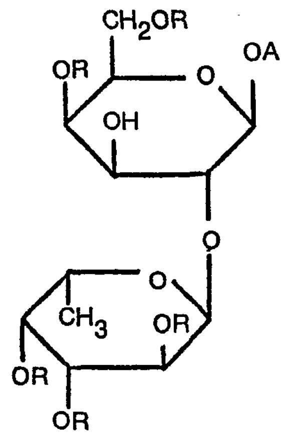

(IV)

in which formulae: the substituents *R*, which are identical or different from one another, represent groups for protecting hydroxyl radicals, these protective groups optionally incorporating a neighbouring substituent, and are chosen from amongst stable groups which are unreactive under the usual conditions of oside synthesis and can readily be removed under mild conditions compatible with the retention of the oside structure, in particular from amongst groups which form, together with the oxygen atom of the hydroxyl, benzyl ethers or benzylidene-acetals, *A* represents an organic radical as defined in any one of Claims 6 and 7, and $R_1$ and $R_2$ have the meanings given in Claim 3.

9. Process for the preparation of the trisaccharide derivative of the formula II, according to Claim 8, characterised in that a digalactopyranose which contains a free group —OH in the 2-position of one of galactopyranose units and corresponds to the formula V:

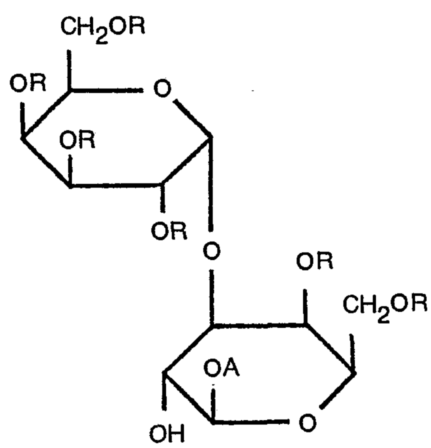

(V)

is employed, and in that it is condensed with the group —O-imidyl of 1-O-imidyl-*β*-*L*-fucopyranose of the formula VI:

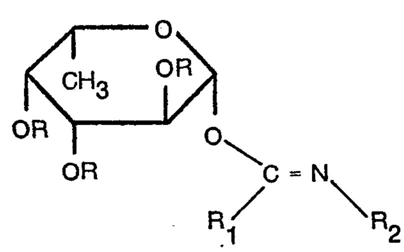

(VI)

$R_1$, $R_2$, *R* and *A* having in these formulae, the meanings given in Claim 8.

10. Process according to Claim 8 or 9, characterised in that the substituent *A* of the disaccharide of the formula IV consists of the allyl radical.

11. Process according to any one of the preceding claims, characterised in that the reaction is carried out in the presence of anhydrous p-toluenesulfonic acid and nitromethane.

12. Application of the process according to any one of Claims 8 to 11, to the synthesis of

trisaccharide B, characterised in that the substituent *A* is sequentially removed, under mild conditions in accordance with the conventional techniques, from the resulting product of the structure II, which leads to a product of the structure IX:

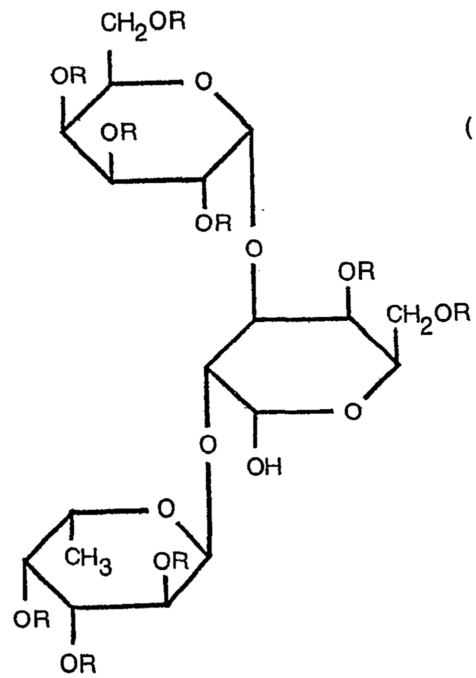

(IX)

and this is followed by hydrogenolysis.

13. New osides as obtained by reacting an oside derivative a) which consists of one or more ose units optionally attached to an organic radical, this ose unit, or at least one of these ose units, being substituted, on the anomeric carbon in the 1-position, by a group —O-imidyl of the formula —O—(—$R_2$)=N—$R_1$, in which the substituents $R_1$ and $R_2$, which are identical or different from one another, represent an aryl radical or an alkyl radical preferably containing 1 to 4 carbon atoms, and the groups —OH of the oside derivative being protected by suitable groups, with an oside derivative b) which consists of one or more ose units optionally attached to an organic radical, only one group —OH of this or these ose units being free and occupying any one of the secondary hydroxyl positions 1 to 4, the oside derivatives a) and/or b) comprising one unit having at least two oses, said osides being characterized by the fact that they contain at least one galactopyranose unit in which the hydrogen atom of the group —OH in the 1-position is substituted by a radical *A* which represents an alkyl group substituted by at least one hydroxyl group which, if necessary, is protected by a blocking group or forms part of a functional radical, or also represents a group containing one or more unsaturated bonds, in particular an ethylenically unsaturated radical chosen from amongst alkenyl radicals, and more especially alkenyl radicals having 2 to 10 carbon atoms or an alkoxy-alkenyl, alkoxy-alcohol, alkoxy-ester, -amide or -amine, alkoxy-alkylamine or alkyl-amide or alkoxy-carboxylic acid radical, these various chemical groups optionally including an intercalary amine function.

14. New osides according to claim 13, wherein *A* represents an alkyl radical containing 2 to 10 carbon atoms, substituted by at least an —OH group which is optionally protected by a blocking group or engaged in a functional radical.

15. New osides according to claim 13 characterised in that the radical *A* represents an allyl radical.

16. New osides according to claim 13 wherein *A* represents a functional group containing at least an ether and/or amine radical.

17. New osides according to anyone of the preceding claims, characterised in that said galactopyranose unit forms part of a linear or branched trisaccharide chain comprising a fucopyranose unit and another galactopyranose unit.

18. New osides according to claim 17, characterised in that they are branched trisaccharides of the formula II:

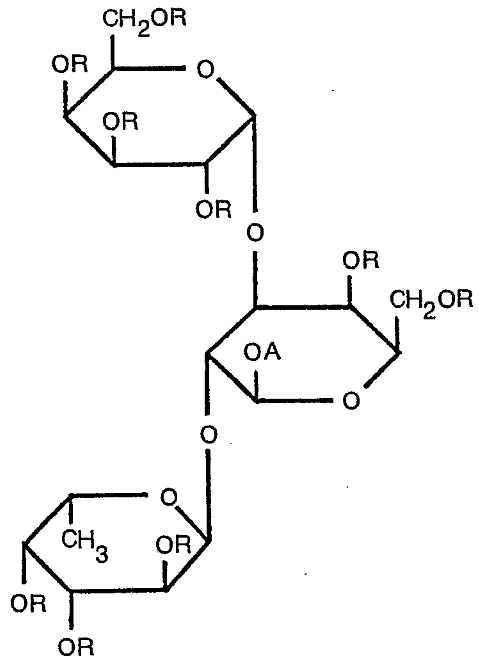

(II)

in which: the substituents *R*, which are identical or different from one another, represent groups for protecting hydroxyl radicals, these protective groups incorporating a neighbouring substituent if desired, and are chosen from amongst stable groups which are unreactive under the usual conditions of oside synthesis and can readily be removed under mild conditions compatible with the retention of the oside structure, and in particular from amongst benzyl or benzyl groups, or represent a hydrogen atom, and *A* represents an organic radical which contains at least one group into which functional groups can be introduced, and which consists, in particular, of an ethylenically unsaturated radical chosen from amongst alkenyl radicals preferably having 2 to 10 carbon atoms, and more particularly consists of the allyl radical, or also consists of a hydroxyalkyl radical, and more especially a $\beta$-hydroxyalkyl radical, $\gamma$-hydroxypropyl, $\gamma$-allyloxypropyl, $\gamma[(\gamma$-hydroxy) · propyloxy] propyle, a $—CH_2—CH_2CH=CH—COOCH_3$ or $—(CH_2)_3—O—(CH_2)_4$ $NH_2$ group, the —OH group (s) being optionally protected or blocked.

19. The trisaccharides according to Claim 18, in which the various substituents *R* represent a hydrogen atom.

20 The trisaccharides of the formula II according to Claim 18, in which *A* is an allyl group.

21. Application of the trisaccharides according to Claim 20 to the synthesis of trisaccharide B in accordance with the process of Claim 12.

22. Biological applications of the osides according to any one of Claims 13 to 20, as immunoabsorbents or for the making of an artificial antigen.

23. Application of the osides according to any one of Claims 13 to 20, in the free form or immobilised on a carrier, to the formation of bioligical reagents, in particular for the detection of the neutralisation of haemolysins.

24. Application of the osides according to any one of claims 18 to 20, in the free form or immobilized on a carrier, to the preparation of testing sera possessing a given specificity.

**Patentansprüche**

1. Verfahren zur Herstellung von Osidderivaten, dadurch gekennzeichnet, daß man

—ein Osidderivat a), welches aus einer oder mehreren Oseeinheiten gelbildet ist, die gegebenenfalls an einen organischen Rest gebunden ist bzw. sind, wobei diese Oseeinheit oder mindestens eine dieser Oseeinheiten am anomeren Kohlenstoffatom in der 1-Stellung mit einer O-Imidylgruppe der Formel $—O—C(—R_2)=N—R_1$ in der die Substituenten $R_1$ und $R_2$, die gleich oder voneinander verschieden sein können, Alkylgruppen mit vorzugsweise 1 bis 4 Kohlenstoffatomen bedeuten, substituiert ist und wobei die OH-Gruppen des Osidderivats durch geeignete Gruppen geschützt sind, mit

—einem Osidderivat b) umsetzt, welches aus einer oder mehreren Oseeinheiten gebildet ist, die gegebenenfalls an einen organischen Rest gebunden ist bzw. sind, wobei eine einzige OH-Gruppe dieser Oseeinheit(en) frei ist und eine der sekundären Hydroxylgruppenstellungen 1 bis 4 besetzt und

wobei die Osidderivate a) und/oder b) eine Einheit aus mindestens zwei Osen aufweisen.

2. Verfahren zur Herstellung von Osidderivaten, dadurch gekennzeichnet, daß man zur Herstellung von verzweigten Osiden das Osidderivat a) nach Anspruch 1 mit einem Osidderivat umsetzt, welches aus einer oder mehreren Oseeinheiten gebildet ist, die gegebenenfalls an einen organischen Rest gebunden ist bzw. sind, wobei eine einzige OH-Gruppe dieser Einheiten frei ist und eine Hydroxylstellung 1 bis 4 oder 6 besetzt, so daß eine verzweigte Osidkette gebildet werden kann.

3. Verfahren nach Anspruch 1 oder 2, dadurch gekennzeichnet, daß das eingesetzte Osidderivat a) aus einer Galactopyranoseeinheit gebildet ist.

4. Verfahren nach Anspruch 3, dadurch gekennzeichnet, daß das Osidderivat a) aus einer Fucopyranoseeinheit gebildet ist.

5. Verfahren nach einem der Ansprüche 1 bis 4, dadurch gekennzeichnet, daß das Osidderivat b) eine Oseeinheit umfaßt, die in der 1-Stellung einen Substituenten der Formel —OA aufweist, der durch eine funktionelle Gruppe, die under den Bedingungen der Osidsynthese nicht reaktiv ist oder nicht reaktiv gemacht worden ist, oder durch eine funktionalisierbare Gruppe gebildet ist, d.h. im Verlaufe oder am Ende der Synthese die Einführung von funktionellen Gruppen ermöglicht.

6. Verfahren nach Anspruch 5, dadurch gekennzeichnet, daß in der Formel des Substituenten —OA A für einen Rest steht, der eine oder mehrere ungesättigte Bindungen aufweist, wie einen ethylenisch ungesättigten Rest, vorzugsweise einen Alkenylrest mit 2 bis 10 Kohlenstoffatomen sowie irgendeinen Rest, der mit Hilfe klassischer organischer Synthesemethoden eine Funktionalisierung der in Rede stehenden ungesättigten Bindung(en) ermöglicht.

7. Verfahren nach Anspruch 5, dadurch gekennzeichnet, daß A eine Alkylgruppe mit 2 bis 10 Kohlenstoffatomen, die durch mindestens eine OH— Gruppe substituiert ist, die gegebenenfalls durch eine Schutzgruppe geschützt ist oder in einer funktionellen Gruppe vorliegt, oder eine Allylgruppe oder eine funktionelle Gruppe, die mindestens einen Etherrest und/oder Aminrest aufweist, darstellt.

8. Verfahren zur Herstellung eines verzweigten Trisaccharidderivats, welches eine Galactopyranose-Galactopyranose-Fucopyranose-Kette aufweist und der Formel II

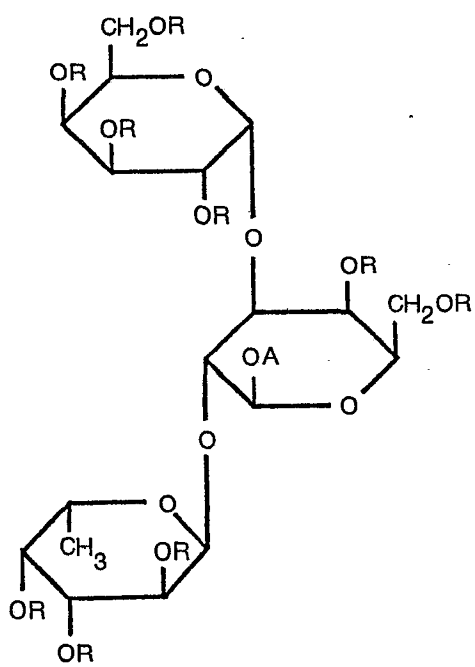

(II)

entspricht, dadurch gekennzeichnet daß man

—eine 1-O-Imidyl-$\beta$-D-galactopyranose der Formel III

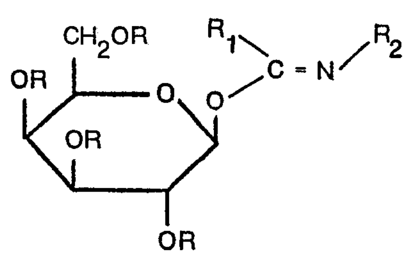

(III)

mit einem Disaccharid, das aus einer Fuco-galactopyranose gebildet ist, die in der 3-Stellung der

Galactopyranoseeinheit eine freie OH-Gruppe aufweist, welches Disaccharid in der Formel IV

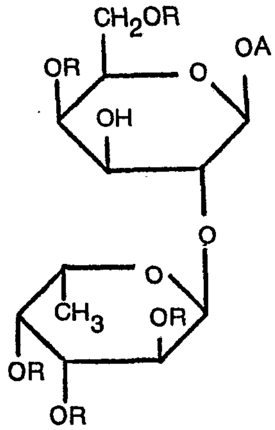

(IV)

entspricht, umsetzt, wobei in den obigen allgemeinen Formeln
—die Substituenten R, die gleichartig oder voneinander verschieden sein können, Schutzgruppen für Hydroxylgruppen gegebenenfalls mit einem benachbarten Substituenten darstellen und aus stabilen Gruppen ausgewählt sind, die unter den üblichen Bedingungen der Osidsynthese nicht reaktiv sind und unter milden Bedingungen, die mit der Aufrechterhaltung der Osidstruktur verträglich sind, leicht abgespalten werden können, insbesondere Gruppen, die mit dem Sauerstoffatom der Hydroxylgruppe Benzylether oder Benzylidenacetale bilden,
—A einen organischen Rest, wie er in den Ansprüche 6 und 7 definiert ist, darstellt und
—$R_1$ und $R_2$ die Bedeutungen besitzen, die in Anspruch 3 angegeben sind.

9. Verfahren zur Herstellung des Trisaccharids der Formel II von Anspruch 8, dadurch gekennzeichnet, daß man eine Digalactopyranose, die eine freie OH-Gruppe in der 2-Stellung einer der Galactopyranoseeinheiten aufweist und die der Formel V

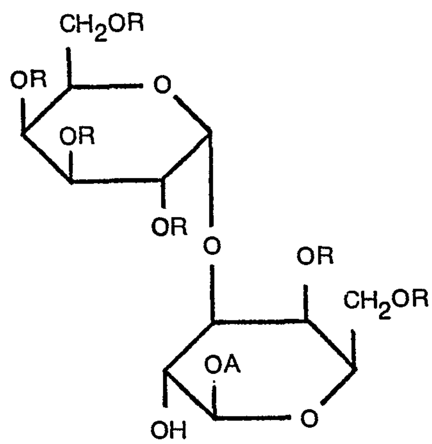

(V)

entspricht, einsetzt und mit der O-Imidylgruppe einer 1-O-Imidyl-β-L-fucopyranose der Formel VI

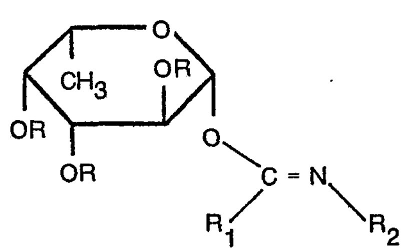

(VI)

kondensiert, wobei die Reste $R_1$, $R_2$, R und A dieser Formeln die in Anspruch 8 angegebenen Bedeutungen besitzen.

10. Verfahren nach Anspruch 8 oder 9, dadurch gekennzeichnet, daß der Substituent A des Disaccharids der Formel IV ein Allylrest ist.

11. Verfahren nach einem dervorhergehenden Ansprüche, dadurch gekennzeichnet, daß man in Gegenwart von wasserfreier p-Toluolsulfonsäure und von Nitromethan arbeitet.

12. Anwendung des Verfahrens nach einem der Ansprüche 8 bis 11 auf die Synthese von Trisaccharid B, dadurch gekennzeichnet, daß man unter Anwendung der milden Bedingungen an sich bekannter Methoden eine sequentielle Eliminierung des Substituenten A des erhalten Produkts der Formel II bewirkt, was zu einem Produkt der Formel IX

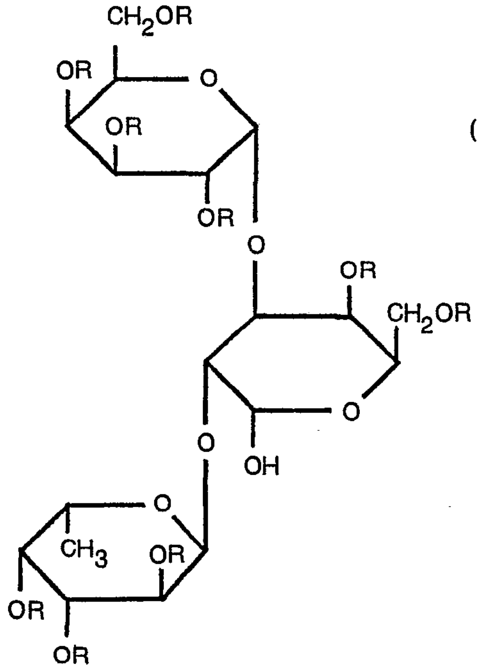

(IX)

führt, welches man einer Hydrogenolyse unterwirft.

13. Neue Oside, erhalten durch Umsetzung

—eines Osidderivats a), welches aus einer oder mehreren Oseeinheiten gebildet ist, die gegebenenfalls an einen organischen Rest gebunden ist bzw. wobei diese Oseeinheit oder mindestens eine dieser Oseeinheiten am anomeren Kohlenstoffatom in der 1-Stellung mit einer —O-Imidylgruppe der Formel —O—C(—$R_2$)=N—$R_1$ in der die Substituenten $R_1$ und $R_2$, die gleichartig oder voneinander verschieden sein können, Allkylgruppe mit vorsugsweise 1 bis 4 Kohlenstoffatomen bedeuten, substituiert ist und wobei die OH-Gruppen des Osidderivats durch geeignete Gruppen geschützt sind, mit

—einem Osidderivat b), welches aus einer oder mehreren Oseeinheiten gebildet ist, die gegebenenfalls an einen organischen Rest gebunden ist bzw. sind, wobei eine einzige OH-Gruppe dieser Oseeinheit (en) frei ist und eine der sekundären Hydroxylgruppenstellungen 1 bis 4 besitzt und wobei die Osidderivate a) und/oder b) eine Einheit aus mindestens zwei Osen aufweisen, wobei diese Oside dadurch gekennzeichnet sind, daß sie mindestens eine Galactopyranoseeinheit aufweisen, in der das Wasserstoffatom der OH-Gruppe in der 1-Stellung durch einen Rest A substituiert ist, der eine Alkylgruppe, die durch mindestens eine Hydroxylgruppe substituiert ist, die gegebenenfalls durch eine Schutzgruppe geschützt oder in einer funktionellen Gruppe vorliegt, oder eine Gruppe, die eine oder mehrere ungesättigte Bindungen aufweist, insbesondere eine ethylenisch ungesättigte Gruppe, die aus der Alkenylgruppen und insbesondere Alkenylgruppen mit 2 bis 10 Kohlenstoffatomen umfassenden Gruppe ausgewählt ist, eine Alkoxyalkenylgruppe, eine Alkoxyalkoholgruppe, ein Alkoxyestergruppe, eine Alkoxyamidgruppe oder eine Alkoxyamin-gruppe, eine Alkoxyalkylamingruppe oder eine Alkylamidgruppe oder eine Alkoxy-carbonsäuregruppe darstellt, welche verschiedenen chemischen Gruppen zusätzlich eine zwischengeschaltete Aminogruppe aufweisen können.

14. Neue Oside nach Anspruch 13, dadurch gekennzeichnet, daß der Rest A eine Alkylgruppe mit 2 bis 10 Kohlenstoffatomen darstellt, die mit mindestens einer OH-Gruppe substituiert ist, die gegebenenfalls durch eine Schutzgruppe geschützt ist oder im Rahmen einer funktionellen Gruppe vorliegt.

15. Neue Oside nach Anspruch 13, dadurch gekennzeichnet, daß A einen Allylrest bedeutet.

16. Neue Oside nach Anspruch 13, dadurch gekennzeichnet, daß A eine funktionelle Gruppe darstellt, die mindestens eine Ethergruppe und/oder Amingruppe aufweist.

17. Neue Oside nach einem der vorhergehenden Ansprüche, dadurch gekennzeichnet, daß die Galactopyranoseeinheit in einer geradkettigen oder verzweigten Trisaccharidkette vorliegt, die eine Fucopyranoseeinheit und eine weiter Galactopyranoseeinheit umfaßt.

18. Neue Oside nach Anspruch 17, dadurch gekennzeichnet, daß sie verzweigte Trisaccharide der Formel II

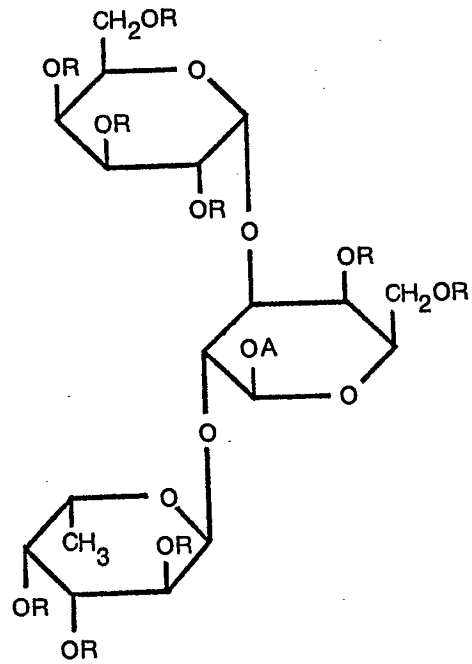

(II)

darstellen, in der

—die Substituenten R, die gleichartig oder voneinander verschieden sein können, gegebenenfalls gemeinsam mit dem benachbarten Substituenten Schutzgruppen von Hydroxylgruppen darstellen, die aus stabilen Gruppen ausgewählt sind, die unter den üblichen Osidsynthesebedingungen nicht reaktiv sind und leicht unter milden Bedingungen, die mit der Aufrechterhaltung der Osidstruktur verträglich sind, abgespalten werden können, insbesondere Benzylgruppen oder Benzylidenylgruppen oder schließlich Wasserstoffatome bedeuten,

—A einen organischen Rest darstellt, der mindestens eine funktionalisierbare Gruppe aufweist, die insbesondere durch einen ethylenisch ungesättigten Rest gebildet wird, der aus Alkenylgruppen mit vorzugsweise 2 bis 10 Kohlenstoffatomen ausgewählt ist und insbesondere ein Allylrest ist oder ein Hydroxylalkylrest, insbesondere ein $\beta$-Hydroxyalkylrest, ein $\gamma$-Hydroxypropylrest, ein $\gamma$-Allyloxypropylrest, ein $\gamma$-[($\gamma$-Hydroxy)-propyloxy]-propylrest, eine Gruppe der Formel $-CH_2-CH_2-CH=CH-COOCH_3$ oder eine Gruppe der Formel $-(CH_2)_3-O-(CH_2)_4-NH_2$ darstellt, wobei die Hydroxylgruppen dieser Reste gegebenenfalls geschützt oder blockiert sind.

19. Trisaccharide nach Anspruch 18, bei denen die verschiedenen Substituenten R Wasserstoffatome bedeuten.

20. Trisaccharide der Formel II nach Anspruch 18, dadurch gekennzeichnet, daß A eine Allylgruppe darstellt.

21. Verwendung der Trisaccharide nach Anspruch 20 bei der Synthese von Trisaccharid B nach dem Verfahren von Anspruch 12.

22. Bioligische Anwendung der Oside nach einem der Ansprüche 13 bis 20 als Immunoabsorbentien oder zur Bildung eines künstlichen Antigens.

23. Anwendung der Oside nach einem der Ansprüche 13 bis 20 in freier Forme oder in immobilisierter Form auf einem Trägermaterial zur Bildung von biologischen Reagenzein, insbesondere zum Nachweis der Neutralisation von Hämolysinen.

24. Anwendung der Oside nach einem der Ansprüche 18 bis 20 in freier Form oder in immobilisierter Form auf einem Träger zur Herstellung von Serum-Tests, die eine gegebene Spezifität besitzen.